# EUROPÄISCHE PATENTANMELDUNG

(11) **EP 0 133 670 A2**
(43) Veröffentlichungstag der Anmeldung: **06.03.1985**
(21) Anmeldenummer: 84108859.4
(22) Anmeldetag: 26.07.1984
(51) Int. Cl.: C07D 501/59, C07D 498/04

(54) **Verfahren zur Herstellung von 7-Acylamino-3-hydrocy-cephem-4-carbonsäuren und 7-Acylamino-3-hydroxy-1-de-thia-1-oxacephem-4-carbonsäuren**

(30) Priorität: 09.08.1983 DE 3328707; 23.05.1984 DE 3419135
(71) Anmelder: BAYER AG, 51368 Leverkusen (DE)
(72) Erfinder: Häbich, Dieter, Dr., D-5600 Wuppertal 1 (DE); Hartwig, Wolfgang, Dr., D-5600 Wuppertal 1 (DE)

(57) **Zusammenfassung**

Die Erfindung betrifft eine Verfahren zur Herstellung von 7.Acylamino-3-hydroxy-cephem-4-carbonsäuren und 7-Acylamino-3-hydroxy-1-dethio-1-oxacephem-4-carbonsäuren und ihrer Derivate der allgemeinen Formel (1) 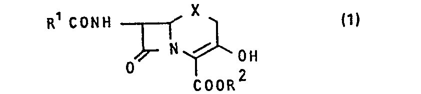 in der R¹, R' und X die in der Beschreibung angegebene Bedeutung haben, durch Umsetzung von Verbindungen der allgemeinen Formel (4) 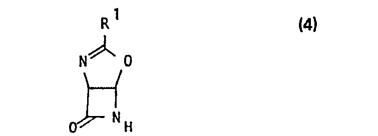 mit einer Verbindung der Formel (5) 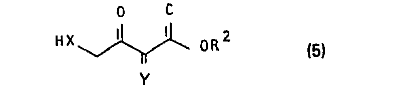 in der R² und X die angegebene Bedeutung haben und Y für Diazo (N₂) oder Wasserstoff (H₂) steht, in einem inerten Lösungsmittel in Gegenwart eines Lewissäure- oder Protonensäurekatalysators.

Gemäß einer Variante des Verfahrens setzt man Verbindungen der Formel (4) mit einer Verbindung der Formel (6) 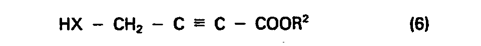 wie oben beschrieben um, hydratisiert die Dreifachbindung der dabei entstehenden Zwischenverbindung und setzt die so für Y = Wasserstoff (für Y = Diazo entstehen direkt die Verbindungen der allgemeinen Formel (2) erhaltenen Verbindungen der allgemeinen Formel (3) 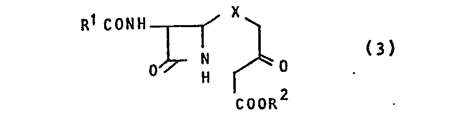 in einem Lösungsmittel mit einem Azid um und bestrahlt oder erwärmt in Gegenwart eines Katalysators die Verbindung der Formel (2) 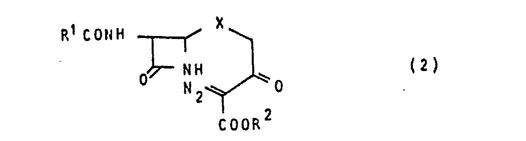 so daß die Verbindungen der allgemeinen Formel (1) entstehen.

## Beschreibung

Die Erfindung stellt ein Verfahren zur Herstellung von 7-Acylamino-3-hydroxy-cephem-4-carbonsäuren und 7-Acylamino-3-hydroxy-cephem-1-dethia-1-oxa- cephem-4-carbonsäuren zur Verfügung.

Das erfindungsgemäße Verfahren zur Herstellung von 7-Acylamino-3-hydroxy-3-cephem-4-carbonsäuren und 7-Acyl- amino-3-hydroxy-1-dethia-1-oxa-3-cephem-4-carbonsäuren und ihrer Derivate der allgemeinen Formel (1), 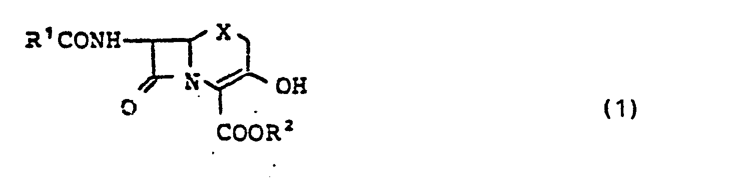 in welcher
R¹ für Wasserstoff oder für gegebenenfalls substituiertₑₛ Alkyl, Alkenyl, Alkinyl, Aralkyl, Aryl, Heteroaryl, Heteroaralkyl, Aryloxyalkyl, Heteroaryloxyal- ₖyₗ, Alkoxyalkyl, Arylthioalkyl, Heteroarylthioalkyl, Alkylthioalkyl, Alkoxy, Aryloxy, Alkylthio oder Arylthio steht,
_{R}² Wasserstoff oder eine Carboxyschutzgruppe oder ₑi-ₙₑn pharmazeutisch verwendbaren Esterrest bedeutet,
_{X} für Schwefel oder Sauerstoff steht,

ist dadurch gekennzeichnet, daß man Verbindungen der allgemeinen Formel (4), 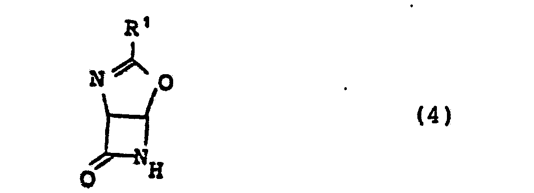 in welcher
R¹ die oben angegebene Bedeutung hat,

a) mit einer Verbindung der allgemeinen Formel (5), 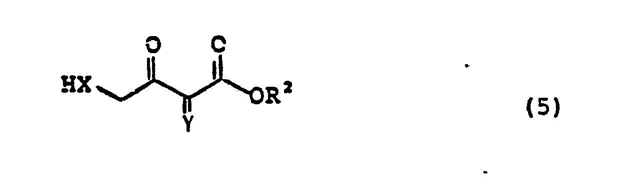 in welcher
   _{R}² und X die oben angegebene Bedeutung haben und Y für Diazo (N₂) oder Wasserstoff (H₂) steht, in einem inerten Lösungsmittel, wie beispielsweise Dichlormethan oder THF, in Gegenwart eines Lewissäure- oder Protonensäurekatalysators umsetzt, oder
b) mit einer Verbindung der allgemeinen Formel (6), 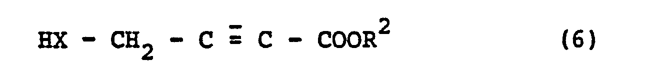 in welcher
   _{R}² und _{X} die oben angegebene Bedeutung haben,

   wie unter a) beschrieben umsetzt und die Dreifachbindung der dabei entstehenden Zwischenverbindung hydratisiert, die so für Y = Wasserstoff (für Y = Diazo entstehen direkt die Verbindungen der allgemeinen Formel (2))erhaltenen Verbindungen der alloemeinen Formel (3), 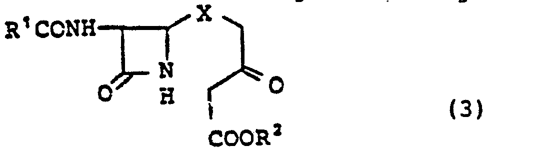 in welcher
   R¹, R² und X die oben angegebene Bedeutung haben,

   in einem Lösungsmittel, wie beispielsweise Acetonitril, mit einem Azid, wie beispielsweise 4-Carboxybenzolsulfonylazid, in Gegenwart einer Base zur Verbindung der allgemeinen Formel (2) umsetzt und die so erhaltenen Verbindungen der allgemeinen Formel (2), 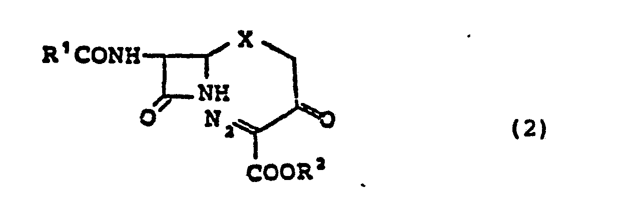 in welcher
   _{R}¹₉ R² und _{X} die oben angegebene Bedeutung haben,

   in einem inerten Lösungsmittel, wie beispielsweise Benzol, durch Bestrahlen oder durch Erwärmen in Gegenwart eines Katalysators, wie beispielsweise Rhodium(II)acetat zu Verbindungen der allgemeinen Formel (1) umsetzt.

Die Verbindungen der allgemeinen Formel (4) können dadurch erhalten werden, daß man
(a) N-substituierte Oxazolinoazetidinone der allgemeinen Formel (7) 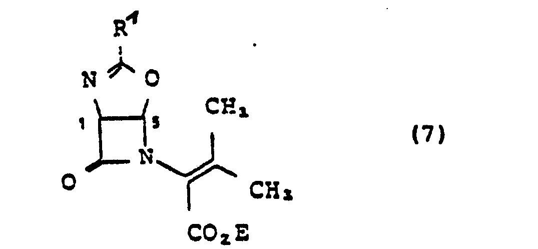 in weicner

R¹ die oben angegebene Bedeutung hat und
   C0₂E für eine Säurefunktion CO₂H oder eine beliebige Esterfunktion steht, wobei E für die in der ß-Lactamchemie üblicherweise verwendeten Säureschutzgruppen steht, vorzugsweise für einen C₁-C₄-Alkylrest steht,
   mit einem Oxidationsmittel unter solvolytischen Bedingungen in einem geeigneten Lösungsmittel oder Lösungsmittelgemisch, gegebenenfalls in Gegenwart von säurebindenden Mitteln oder anschließendem Einsatz von Reduktionsmitteln umsetzt
   oder
(b) Oxamide der allgemeinen Formel (8) 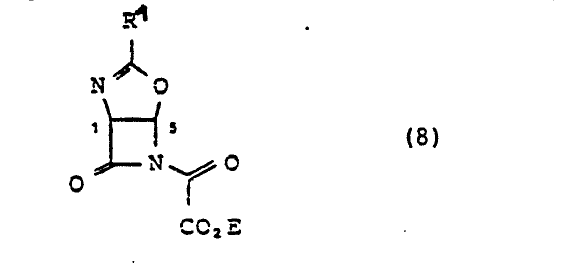 in welcher
   Rund CO₂E die oben angegebene Bedeutung haben, unter solvolytischen Bedingungen in einem geeigneten Lösungsmittel oder Lösungsmittelgemisch, gegebenenfalls in Gegenwart von Säuren oder Basen umsetzt.

Die Verbindungen der allgemeinen Formel (7), in welcher R¹ und CO₂E die angegebene Bedeutung haben, können in Analogie zu bekannten Verfahren dadurch erhalten werden, daß man die olefinische Bindung von Verbindungen der allgemeinen Formel (9) 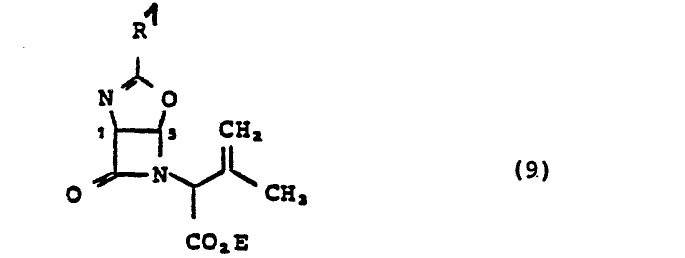 in welcher .
R¹ und CO₂E die oben angegebene Bedeutung haben,

in einem geeigneten Lösungsmittel in Gegenwart von Base einer Isomerisierung unterwirft, wie sie beispielsweise bei Y.Maki et al., J.C.S. Perkin I (1981) 2087, Y. Hamashima et al., Tetrahedron Lett. (1979) 2595, S. Uyeo et al., J. Am. Chem. Soc. 101, 4403 (1979) und in der Belgischen Patentschrift 862 793 beschrieben ist.

Die _{O}xazolinooxamide der allgemeinen Formel (8) können ebenfalls in Analogie zu bekannten Verfahren dadurch erhalten werden, daß man die olefinische Bindung von _{A}ze- tidinonen der allgemeinen Formel (7), in welcher R¹ und C0₂E die angegebene Bedeutung haben, einer oxidativen Spaltung unterwirft wie sie beispielsweise in der Deutschen Offenlegungsschrift 28 39 646, der Europäischen Patentschrft 21 676, der Belgischen Patentschrift 849 118 und bei S. Yamamoto et al. Heterocycles 8, 282 (1977) und M. Narisada et al. J. Med. Chem. 22, 757 (1979), Heterocycles 7, 839 (1977) in analoger Weise beschrieben ist.

Außerdem können die Verbindungen der allgemeinen Formel (7) auch in Analogie zu anderen literaturbekannten Verfahren hergestellt werden, wie z.B. in der Japanischen Patentschrift 55 047 687, der Niederländischen Patentschrift 7 313 896 oder bei Y. Hamashima et al., Tetrahedron Lett. (1979) 4943 beschrieben.

Als mögliche Ausgangsprodukte für optisch aktive Oxazolinoazetidinone der allgemeinen Formel (1S, 5R) (4) und (1R,5S) (4) werden gegebenenfalls die entsprechend konfugierten Verbindungen der allgemeinen Formel (9) verwendet.

Für die Synthese beider Enantiomere der Verbindungen der allgemeinen Formel (9), in der R¹ = Phenyl ist, existieren Literaturbeispiele:
(1S,5R) (9): S. Yamamoto et al., Tetrahedron Lett. (1981) 3089,
(1R,5S) (9): Y. Hamashima et al., Tetrahedron Lett. (1979) 2595.

Andere Verbindungen (9) können in analoger Weise hergestellt werden.

Verwendet man z.B. Methyl-2-[(1S,5R)-3-benzyl-7-oxo-4-oxa-2,6-diazabicyclo[3.2.0]hept-2-en-6-yl)]-3-methyl- but-3-enoat (9a), so kann der Reaktionsablauf zur Herstellung der Verbindungen (4) durch das folgende Formelschema wiedergegeben werden: 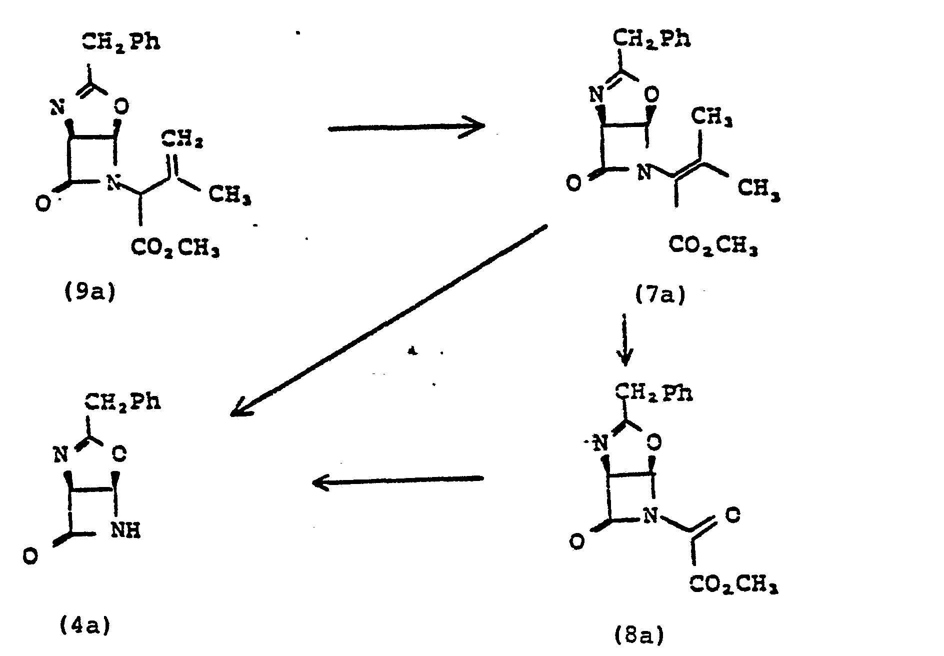 Bei der Umsetzung von Verbindungen der Formel (9) zu Verbindungen der Formel (7) kommen als Reagentien alle üblichen organischen und anorganischen Basen in Betracht. Hierzu gehören vorzugsweise die Alkalihydroxide, Alkalicarbonate, Alkaliamide und organische Amine. Besonders geeignet sind Kaliumcarbonat, Triethylamin, Diisopropylethylamin, Pyridin, Dimethylanilin, Diethylamin, 1,5-Diazabicyclo(5,4,0)undec-5-en (DBU), bzw. DBN und Ammoniak.

Als Verdünnungsmittel kommen alle inerten organischen Lösungsmittel sowie organische Basen und Wasser in Betracht. Hierzu gehören vorzugsweise Ethylacetat, Tetrahydrofuran, Dichlormethan, Dichlorethan, Dichlorbenzol, Toluol, Ethylamin und Dimethylamin. Die Isomerisierung erfolgt im allgemeinen bei Temperaturen zwischen -50 und +50°C, vorzugsweise jedoch zwischen 0°C und Raumtemperatur.

Bei der Umsetzung von Verbindungen der Formel (7) zu Verbindungen der Formel (8) kommen als Reagentien alle üblichen Oxidationsmittel in Betracht, die eine olefinische Doppelbindung in der angegebenen Weise zu spalten vermögen.

Vorzugsweise seien Natriumperiodat, Osmiumtetroxid, Sauerstoff-Ozon sowie deren Gemische genannt. Als Verdünnungsmittel kommen alle inerten organischen Lösungsmittel in Frage. Hierzu gehören vorzugsweise Ethylacetat, Ethanol, Methanol, Tetrahydrofuran, Dichlormethan, Dichlorethan, Toluol und Dioxan bzw. Gemische derselben.

Gegebenenfalls ist bei diesem Reaktionsschritt vor der Aufarbeitung ein Reduktionsmittel zuzusetzen. Vorzugsweise können dafür anorganische bzw. organische Schwefelverbindungen eingesetzt werden. Besonders geeignet sind Diorganylsulfide wie z.B. Dimethylsulfid.

Die Umsetzungen erfolgen im allgemeinen zwischen -80°C und +50°C bevorzugt jedoch zwischen -80°C und 0°C.

Bei der Umsetzung von Verbindungen der Formel (7) zu den Verbindungen der Formel (4) kommen als Reagentien alle üblichen Oxidationsmittel in Frage. Bevorzugt finden solche Verwendung, die zunächst zu einer Dihydroxylierung der konjugierten Doppelbindung führen. Die erwähnte Umsetzung kann gegebenenfalls in Analogie zu bekannten Verfahren durchgeführt werden (E.G. Brain et al., J.C.S. Chem. Comm. (1972) 229, Deutsche Offenlegungsschrift 21 56 352, A.K. Bose et al., Tetrahedron 37, 2321 (1981), J.S. Wells et al., J. Antibiot. 35 189 (1982)).

Bevorzugte Oxidationsmittel sind Kaliumpermanganat, Natriumperiodat, Osmiumtetroxid und deren Gemische.

Ebenso können Oxidationsmittel wie Bleitetraacetat, Kupfer(TI)acetat oder N-Halogensuccinimide und -phthalimide verwendet werden. Als Lösungsmittel kommen sämtliche Lösungsmittel in Frage, welche solvolytisch wirksam sind und die derartige Ablösung des oxidierten Butenoat-Restes von ß-Lactam bewirken. Im besonderen seien genannt: Wasser, Methanol, Ethanol, Aceton, Pyridin, Triethylamin, DMF oder Gemische derselben. Gegebenenfalls können basische oder saure Hilfsmittel verwendet werden. Hierzu gehören vorzugsweise Kaliumcarbonat, Puffer-Lösungen, organische Amine wie Triethylamin oder Pyridin, Schwefelsäure, Kieselsäure bzw. Kieselgel, oder organische Sulfonsäuren. Die Umsetzung erfolgt bevorzugt zwischen -40°C und +80°_{C}.

Bei der Umsetzung von Verbindungen der Formel (8) zu den Verbindungen der Formel (4) kommen als Verdünnungssämtliche solvolytisch wirksamen Lösungsmittel in Frage, die geeignet sind die Solvolyse der Oxamidstruktur zu bewirken, wie sie analog beispielsweise in der Europäischen Patentschrift 21 676, der Deutschen Offenlegungsschrift 28 39 646 und bei R.D.G. Cooper et al., J. Am. Chem. Soc. 94, 1021 (1972) beschrieben ist.

Bevorzugt gehören hierzu organische Alkohole, primäre Amine und Wasser bzw. deren Mischungen mit inerten Lösungsmitteln. Besonders erwähnt seien Methanol, und weitere Alkohole mit 1-5 C-Atomen. Gegebenenfalls können zur Unterstützung der Umsetzung basische oder saure Hilfsmittel zugesetzt werden. Hierzu gehören vorzugsweise Alkalialkoholate, Alkalicarbonate, Puffer-

lösungen, organische Amine, Carbonsäuren, Sulfonsäuren und anorganische Protonensäuren. Besonders seien erwähnt: Natriummethanolat, Kaliumcarbonat, schwach basische bzw. schwach saure Pufferlösungen, Schwefelsäure, Perchlorsäure, Phosphorsäure, Kieselsäure und Kieselgel. Die Umsetzung erfolgt bevorzugt zwischen -30°C und +70°C besonders jedoch zwischen 0°C und Raumtemperatur. ,

Verbindungen der allgemeinen Formel (5) in welcher X und R² die angegebene Bedeutung haben und Y für Wasserstoff (H₂) steht,können in Analogie zu bekannten Verfahren (Europäische Patentschrift 67 409; K. Clauss, Liebigs Ann. Chem. 494 (1980)) aus 4-Halogen-acetessigestern hergestellt werden: 4-Halogen-acetessigsäureester lassen sich in bekannter Weise mit 0- und S-Nucleophilen umsetzen. Die benötigten 4-Hydroxy- und 4-Mercapto-acetessigester können durch Abspaltung der betreffenden Schutzgruppen freigelegt werden (T.W. Greene, Protective Groups in Organie Syntheses, J. Wiley & Sons (1981)).

Verbindungen der allgemeinen Formel (5), in welcher X und R² die angegebene Bedeutung haben und Y für Diazo (N₂) steht, können in Analogie zu bekannten Verfahren (R.W. Ratcliffe et al. Tetrahedron Lett. (1980) 31; US-Patentschrift 4 310 538) durch Diazotransferreaktionen aus Verbindungen der allgemeinen Formel (5), in welcher X und R² die angegebene Bedeutung haben und Y für Wasserstoff (H₂) steht, hergestellt werden.

Die Verbindungen der allgemeinen Formel (6) können nach literaturbekannten Verfahren oder in Analogie dazu hergestellt werden [R.A.Earl et al., Organic Syntheses 60, 81 (198117. Methoden zur Hydratisierung von Dreifachbindungen in der ß-Lactamchemie sind ebenfalls bekannt [S.Ikegami et al., Tetrahedron Lett. 2875 (198217.

Ein Merkmal der Erfindung ist die stereoselektive Ringöffnung N-unsubstituierter Oxazolinoazetidinone der allgemeinen Formel (4) mit Nucleophilen der allgemeinen Formeln (5) bzw. (6).

Es ist ausgesprochen überraschend zu bezeichnen, daß diese Umsetzung an N-unsubstituierten Oxazolinoazetidinonen durchgeführt werden kann. Durch den nicht von vornherein anwesenden N-Substituenten wird das Spektrum der möglichen Zielverbindungen entscheidend erweitert. So können erfindungsgemäß im Gegensatz zum Stand der Technik, sowohl mono- als auch bifunktionelle Reagentien eingesetzt werden, welche im Anschluß die Annelierung von Ringen an das Azetidinon ermöglichen.

Ein weiteres Merkmal der Erfindung ist die Annelierung von schwefel- oder sauerstoffhaltigen 6-Ringen an das Azetidinon durch intramolekulare Einschubreaktion eines aus Diazoessigestern erhaltenen Carbens in die N-H-Bindung des Azetidinons.

Solche Anellierungsreaktionen unter Bildung von 6-Ringen sind für X = CH₂ (U.S. Patent 4.174.316; T. N. Salzmann et al. Tetrahedron Lett. (1980) 1193) und für X = O (EP .81824) bekannt geworden. Für X = S ist dieser Reaktionstyp noch nicht beschrieben.

Die erfindungsgemäße Verfahrensfolge weist eine Reihe von Vorteilen auf. Die erfindungsgemäße Synthese ist konvergent und damit effektiv - d.h. in der Art eines Baukastenprinzips werden zwei fertige Molekülteile vereinigt.

Alle heute bekannten Verfahren zur Herstellung von 3-Hydroxy-Cephalosporin- bzw. Oxacephemderivaten sind vielstufige lineare Synthesen, bei denen sämtliche Reaktionsschritte in Gegenwart des vorgefertigten ß-Lactamrings erfolgen, was angesichts der bekannten Empfindlichkeit des ß-Lactamrings von entscheidendem Nachteil ist. Lineare Synthesen beinhalten gegenüber konvergenten Synthesen außerdem einen deutlichen strategischen Nachteil (S. Warren, Designing Organic Syntheses, J. Wiley + Sons, 1978, S. 201).

Bausteine der allgemeinen Formel (4) lassen sich je nach Wahl des Reaktionspartners zu einer Vielzahl denkbarer bicyclischer ß-Lactamverbindungen umsetzen. Durch diese Strategie bleibt die Anzahl der in Gegenwart des empfindlichen B-Lactamrings durchzuführenden Operationen auf ein Minimum beschränkt.

Verwendet man z.B. (1R,5S)-3-Phenyl-4-oxa-2,6-diazabi- cyclo[3.2.0]hept-2-en-7-on und 4-Mercaptoacetessigsäure- tert.-butylester als Ausgangsmaterialien, so läßt sich der Reaktionsablauf durch das folgende Formelschema wiedergeben:

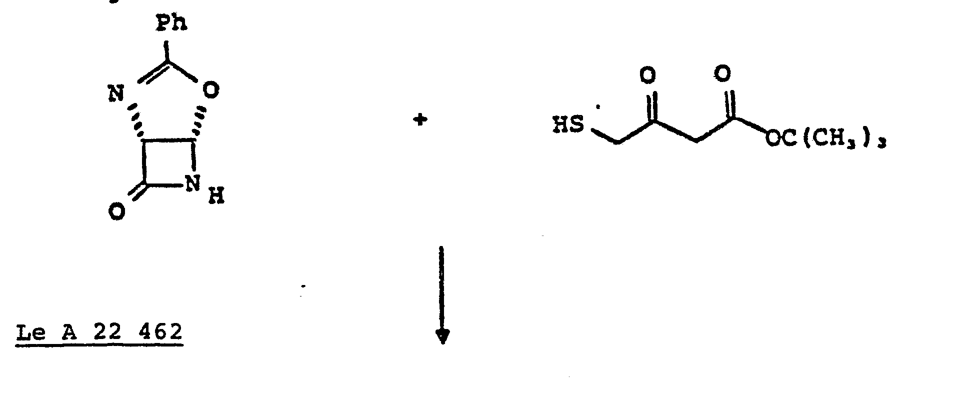

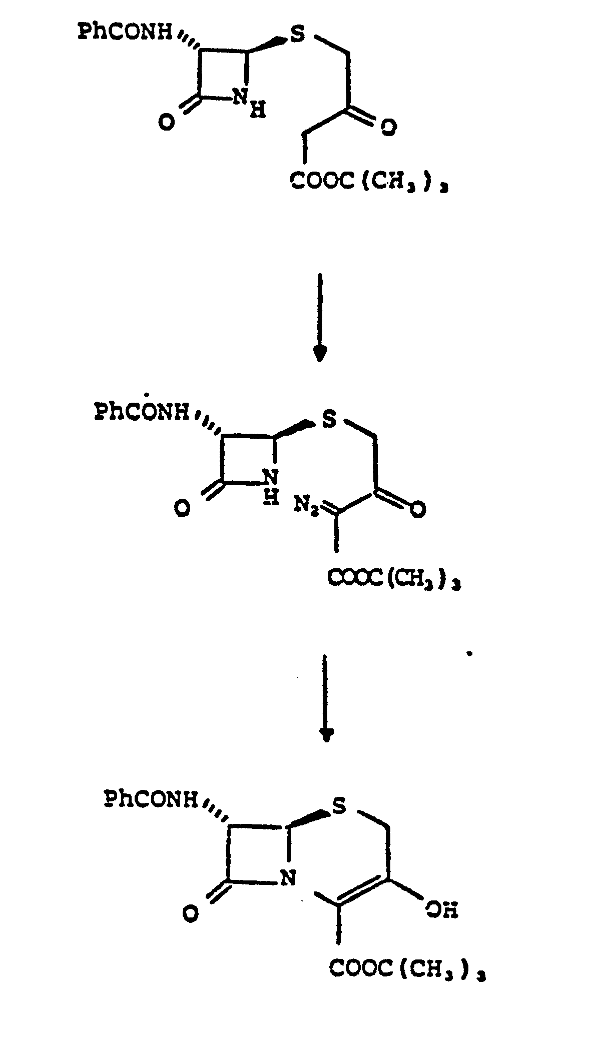

Verwendet man zum Beispiel (1R,5S)-3-Phenyl-4-oxa-2,6-diazabicyclo[3.2.0]hept-2-en-7-on und 4-Hydroxy-2-bu- tinsäure-4-nitrobenzylester als Ausgangsmaterialien, so läßt sich der Reaktionsablauf durch das folgende Formelschema wiedergeben:

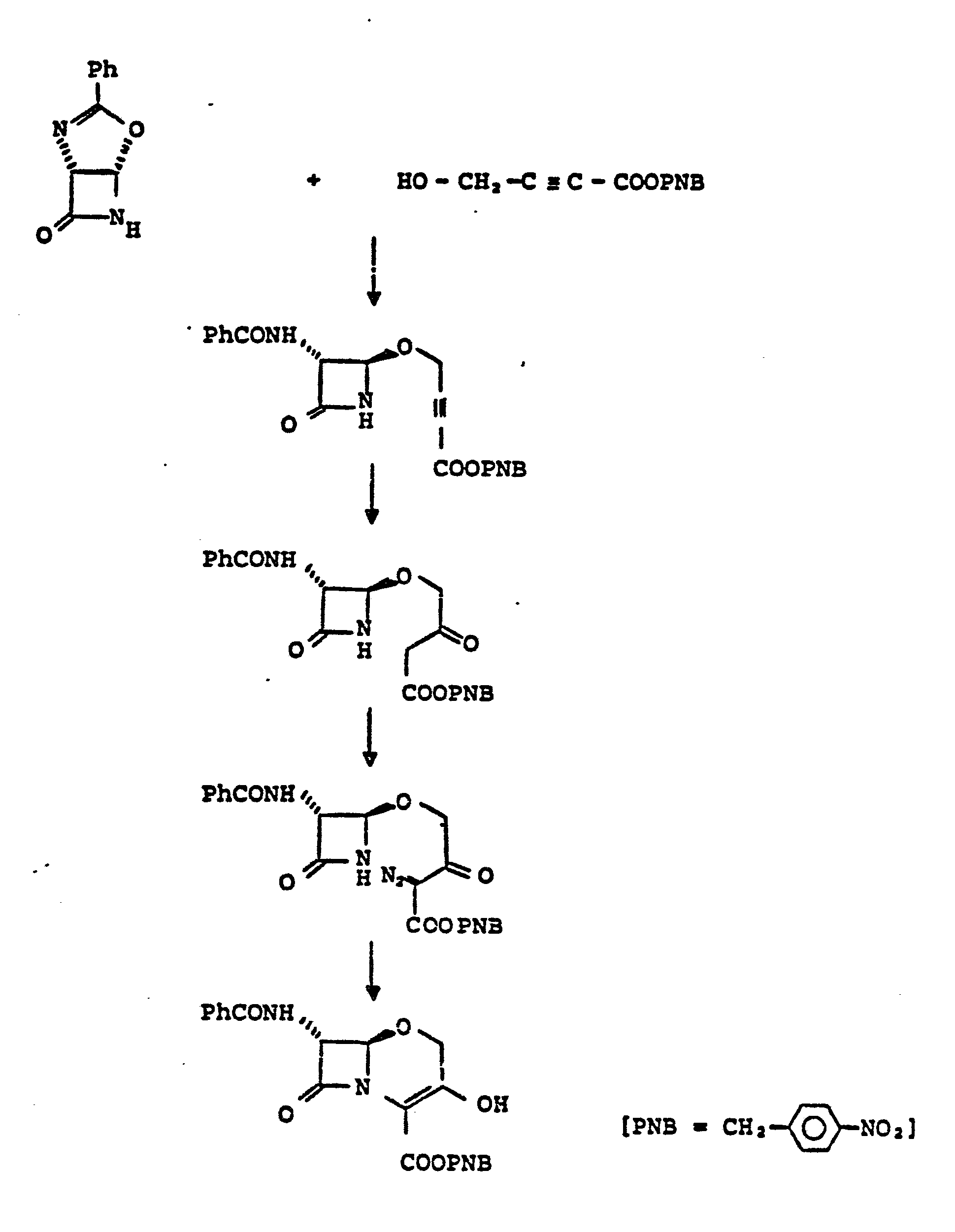

Verwendet man z.B. (lR,SS)-3-Phenyl-4-oxa-2,6-diazabicyclo [3.2.0]hept-2-en-7-on und tert.-Butyl-2-diazo-4-hydroxy-3-oxo-butanoat als Ausgangsmaterialien, so läßt sich der Reaktionsablauf durch das folgende Formelschema wiedergeben:

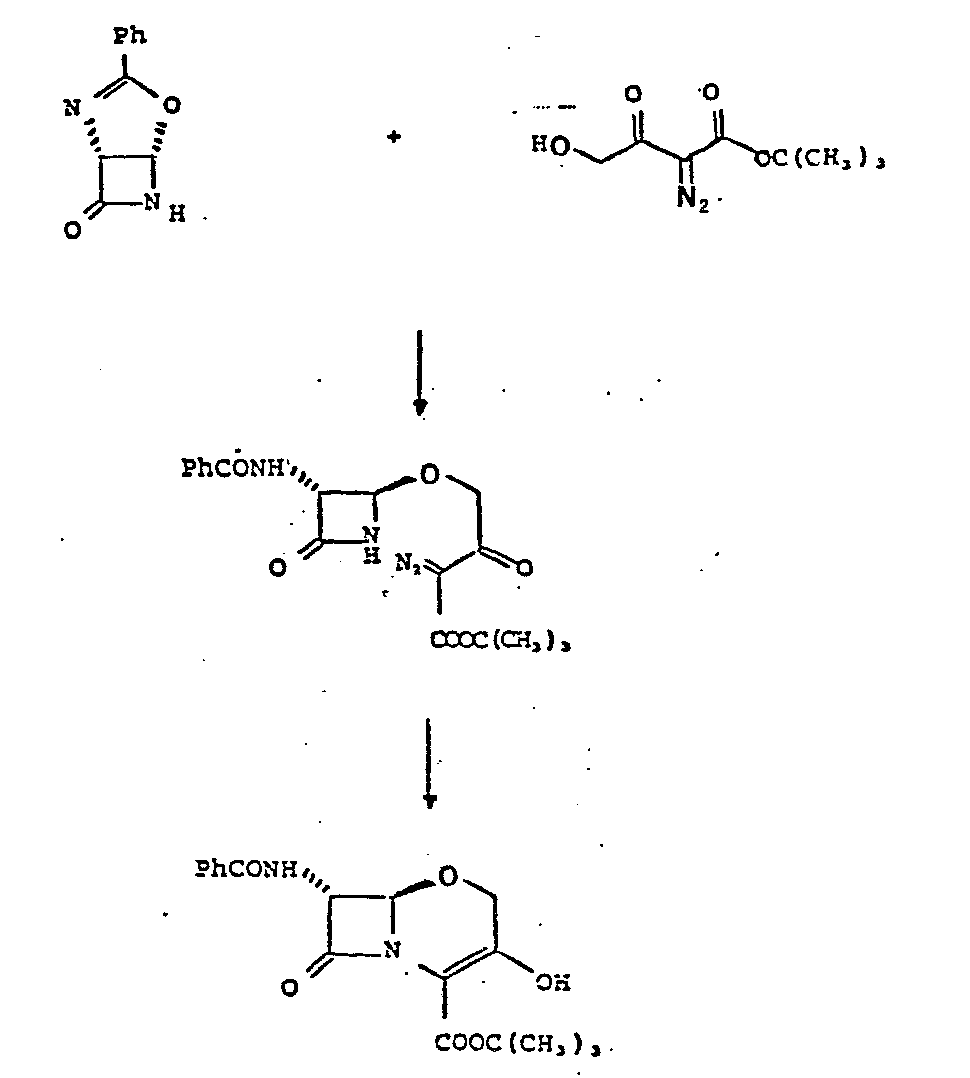

In den Verbindungen der allgemeinen Formeln (1), (2), (3), (4), (7), (8) und (9) steht

R¹ in der Bedeutung von gegebenenfalls substituiertes Alkyl für einen geradkettigen, verzweigten oder cyclischen Kohlenwasserstoffrest mit vorzugsweise 1-7 C-Atomen. Die Alkylreste können gegebenenfalls ungesättigt vorliegen und durch Halogen vorzugsweise Chlor, _{H}ydroxy, Amino, Carboxy, Carbamoyl, Mesyl oder durch im folgenden näher definiertes, gegebenenfalls substituiertes Aryl bzw. Heteroaryl, einfach oder doppelt substituiert sein. Besonders erwähnt seien hier die Reste wie Methyl, Halogenmethyl, tert.-Butyl, Cyclohexyl und Cyclohexadienyl.

_{R}¹ in der Bedeutung von gegebenenfalls substituiertes Aryl steht vorzugsweise für Phenyl, welches durch Methyl, Ethyl, Aminomethyl, Hydroxy, Methoxy, Ethoxy, Carbamoyloxy, Acetoxy, Amino, Mesylamino, Methylamino, Aminosulfonylamino, Amidino, Mesyl, Methylsulfinyl, Methoxycarbonyl, Carbamoyl, Sulfo, Methylthio, Silyl, Silyloxy oder Halogen, vorzugsweise Chlor oder Brom, vorzugsweise einfach oder doppelt, gegebenenfalls aber auch 3- fach substituiert sein kann.

Für gegebenenfalls substituiertes Aralkyl stehen die Kombinationen der unter Aryl und Alkyl genannten Bedeutungen. Besonders erwähnt seien: Benzyl, p-Hydroxybenzyl, p-Aminobenzyl, α -Aminobenzyl, α ,4-Diaminobenzyl, α-Amino-4-hydroxybenzyl , α-Carboxy-benzyl, α-Carboxy-4-hydroxybenzyl und bis-(Trimethylsilyl)-geschütztes α-Carboxy-4-hydroxybenzyl.

Für gegebenenfalls substituiertes Heteroaryl stehen alle im Ring Sauerstoff-, Stickstoff- und/oder Schwefelatome enthaltenden ungesättigten 5- oder 6-gliedrigen Heterocyclen mit 1-4 Heteroatomen, die unsubstituiert oder vorzugsweise durch Methyl, Ethyl, Hydroxy, Oxo, Amino, Imino, Mesyl, Mesylamino, Silyl, Carboxy, Carbamoyl, Acetyl oder Halogen, vorzugsweise Chlor oder Brom, monodi- oder trisubstituiert sein können.

Vorzugsweise steht für einen ungesättigten gegebenenfalls substituierten heterocyclischen Ring die Furyl-, Methylfuryl-, Thienyl-, Methylthienyl-, 2-Aminothiazolyl-, Thiazolyl-, Methylisoxazolyl-, Isoxazolyl-, Pyridyl-, 2-Aminopyridyl-, Pyrimidyl-, Pyrazolyl-, Uracyl-, Thiadiazolyl-, Tetrazolyl- oder Pyranylgruppe.

Für gegebenenfalls substituiertes Heteroalkyl stehen die Kombinationen der unter Alkyl und Heteroaryl vorzugsweise genannten Bedeutungen. Besonders erwähnt seien hier Furylmethyl, Thienylmethyl, 2-Aminothiazolylmethyl, Thiazolylmethyl, Aminopyridylmethyl, 1-Methyl-1-H-tetrazol-5-yl-thiomethyl, 2-Aminothiazolylmethoxyiminomethyl, 1-(2-Aminothiazolyl)-1-propenyl.

Für gegebenenfalls substituiertes Aryloxyalkyl, Heteroaryloxyalkyl und Alkoxyalkyl stehen die obengenannten Bedeutungen, die im Alkylteil oder zwischen Alkyl- und Aryl- bzw. Heteroarylteil eine Sauerstoffbrücke in Form einer Etherfunktion tragen. Besonders erwähnt seien: Phenoxymethyl, 4-Hydroxyphenoxymethyl, α -Aminophenoxymethyl, α-Amino-4-hydroxy-phenoxymethyl, Methoxymethyl, tert.-Butoxymethyl, Thienyloxymethyl, α-Aminothienyloxymethyl, Furyloxymethyl und α-Aminofuryloxymethyl.

Gegebenenfalls substituiertes Alkoxy bzw. Aryloxy steht für oben definierte Alkyl- bzw. Arylreste, welche über eine Sauerstoffbrücke direkt gebunden sind. Besonders erwähnt seien: Methoxy, Ethoxy, tert.-Butoxy, Phenoxy, Benzyloxy, Diphenylmethyloxy, 4-Nitrobenzyloxy und 4-Methoxybenzyloxy.

Gegebenenfalls substituiertes Alkylthio bzw. Arylthio steht für oben definierte Alkyl- bzw. Arylreste, welche über eine Schwefelbrücke direkt gebunden sind. Besonders erwähnt seien: Methylthio, Ethylthio, tert.-Butylthio, Phenylthio, Benzylthio, Diphenylmethylthio und 4-Nitrobenzylthio.

Für gegebenenfalls substituiertes Arylthioalkyl, Heteroarylthioalkyl und Alkylthioalkyl stehen die oben genannten Bedeutungen, die im Alkylteil oder zwischen Alkyl- und Aryl- bzw. Heteroarylteil eine Schwefelbrücke in Form einer Thioetherfunktion tragen. Besonders erwähnt seien: Phenylthiomethyl, 4-Hydroxyphenylthiomethyl, α-Aminophenylthiomethyl, 2-Methyl-1-thia-3,4-diazol-5-yl-thiomethyl, Methylthiomethyl und tert.-Butylthiomethyl.

Beispielhaft seien die nachfolgenden Reste R¹ erwähnt:

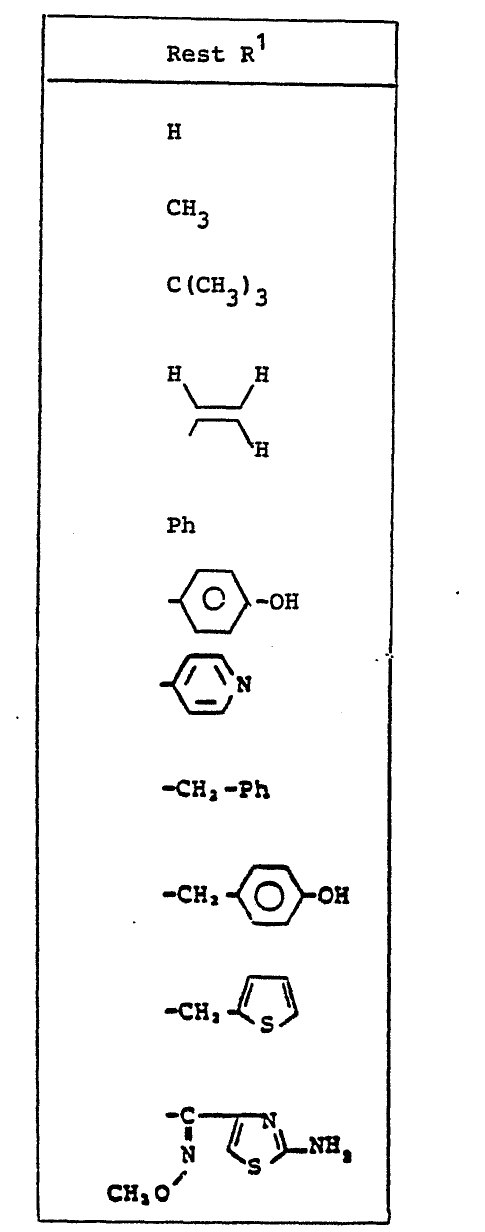

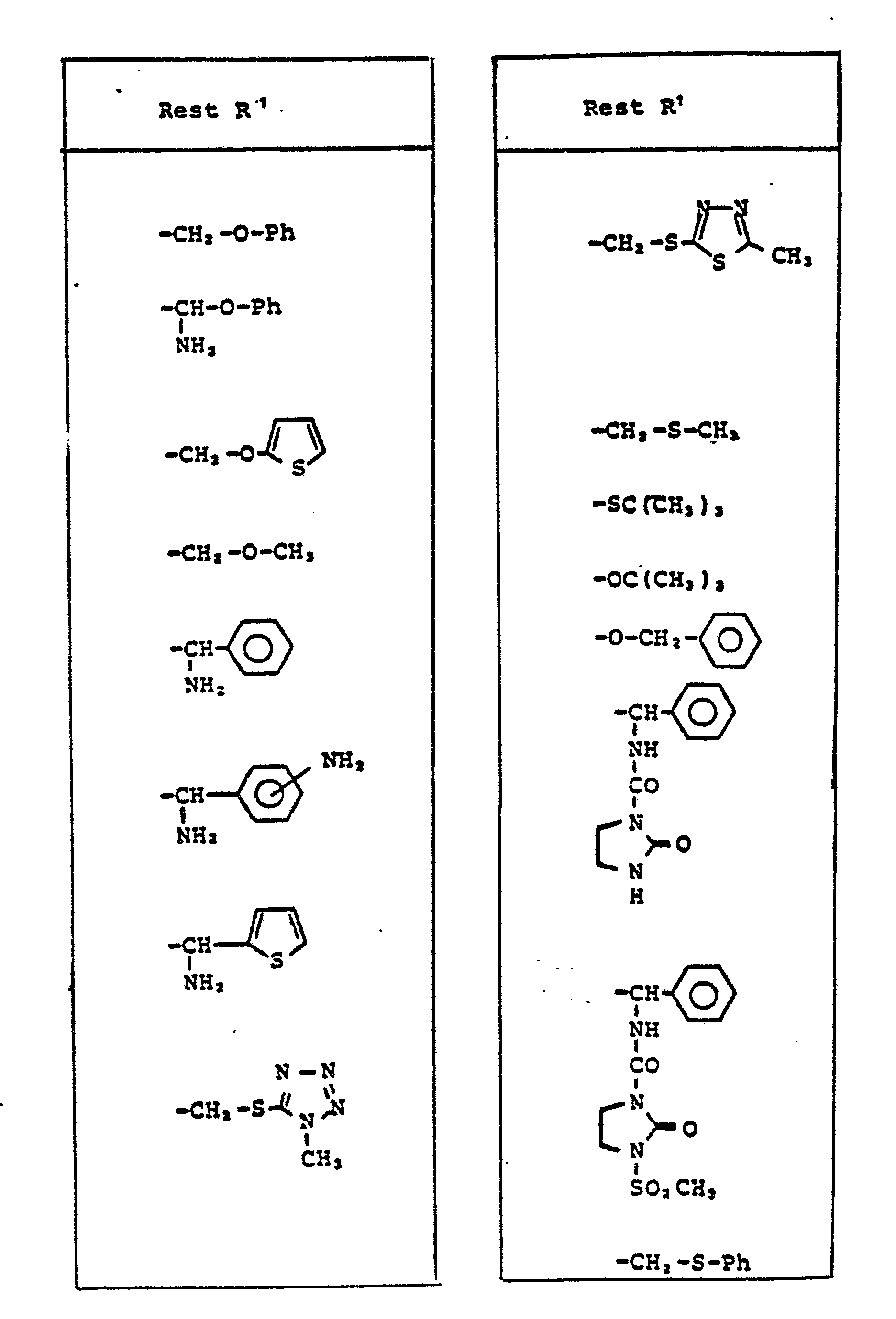

In Verbindungen der allgemeinen Formeln (1), (2), (3), (5) und (6), steht
in der Bedeutung von R² der Ausdruck Carboxyschutzgruppe für die in der B-Lactamchemie bekannten Schutzgruppen, beispielsweise eine gegebenenfalls substituierte Benzyl-, Diphenylmethyl- oder Triphenylmethylgruppe, eine Silylgruppe oder eine gegebenenfalls substituierte Ethyl-oder Allylgruppe. Besonders erwähnt seien Gruppen wie Benzyl, 2-Nitrobenzyl, 4-Nitrobenzyl, 4-Methoxybenzyl, 2,4-Dimethoxybenzyl, Diphenylmethyl, Triphenylmethyl, Trichlorethyl, Chlorethyl, Cyanoethyl, Trimethylsilylethyl und Dimethylethyl.

Steht _{R}² für einen pharmazeutisch verwendbaren Esterrest, so sind hiermit bevorzugt verträgliche Esterreste gemeint, die in vivo leicht zu freien Carboxylgruppen (R² = H) gespalten werden.

Solche Esterreste R² sind auf dem B-Lactamantibiotikagebiet gut bekannt. In den meisten Fällen verbessern sie die Absorptionseigenschaften der B-Lactamverbindung. Außerdem sollte R² von solcher Art sein, daß er einer Verbindung der Formel (1) pharmazeutisch annehmbare Eigenschaften verleiht und bei Spaltung in vivo pharmazeutisch annhembare Fragmente freisetzt. Beispiele solcher Gruppen R² finden sich in der DE-OS 2 517 316.

Bei der erfindungsgemäßen Herstellung von Verbindungen der allgemeinen Formel (1) aus Verbindungen der allgemeinen Formel (2) kommen als Katalysatoren Komplexe oder Salze von Übergangsmetallen oder elementare übergangsmetallen in Betracht. Bevorzugte Katalysatoren sind Komplexe von Kupfer, Rhodium und Palladium, wie beispielsweise [Cu(acac)₂], Rh₂(OAc)₄ und Pd(OAc)₂, außerdem CuS0₄ und Cu-Pulver. Besonders erwähnt sei Rhodium-(II)acetat [Rh₂(OAc)₄]. [acac = Acetylacetonat; OAc = Acetat7.

Im allgemeinen können bis zu molaren Mengen des Katalysators eingesetzt werden. Bevorzugt werden jedoch Katalytische Mengen zwischen 0,1 und 5 Mol-% verwendet.

Als Verdünnungsmittel kommen alle inerten organischen Lösungsmittel in Betracht. Hierzu gehören vorzugsweise aromatische Lösungsmittel wie Benzol, Toluol etc., jedoch auch THF und Dichlormethan. Die Umsetzung erfolgt im allgemeinen bei Temperaturen zwischen 40 und 120°C, vorzugsweise jedoch zwischen 70 und 90°C innerhalb von 0,5-5 h.

Anstelle der Verwendung von Katalysatoren kann die erfindungsgemäße Umsetzung auch durch Bestrahlen von Verbindungen der allgemeinen Formel (2) mit einer Lichtquelle der Welleniänge ≥ 300 nm erfolgen. Als Lösungsmittel kommen hierbei alle inerten organischen Lösungsmittel in Betracht. Hierzu gehören vorzugsweise Benzol, Toluol, Tetrachlormethan, Dichlormethan, Ether, THF und Dioxan. Die Photocyclisierung erfolgt im allgemeinen zwischen -20 und +30°C innerhalb von 0,5 bis 3 h.

Bei der erfindungsgemäßen Herstellung von Verbindungen der allgemeinen Formel (2) aus Verbindungen der allgemeinen Formel (3) kommen als Reagentien alle Azide in Betracht, die in der Lage sind, die erfindungsgemäße Umsetzung zu bewirken. Hierzu gehören beispielsweise gegebenenfalls substituierte Methan-, Benzol- oder Naphthalinsulfonylazide. Besonders erwähnt seien Toluolsulfonylazid, 4-Dodecylbenzolsulfonylazid und 4-Carboxybenzolsulfonylazid. Als Basen kommen alle üblichen anorganischen und organischen Basen in Betracht. Hierzu ge-. hören vorzugsweise Alkalicarbonate, Alkaliamide und organische Amine. Besonders geeignet sind Triethylamin, Diethylamin, Diisopropylethylamin, Pyrimidin, Dimethylanilin, 1,5-Diazabicyclo[5.4.0]undec-5-en (DBU) bzw. DBN.

Als Verdünnungsmittel kommen alle inerten organischen Lösungsmittel und organischen Basen in Betracht. Hierzu gehören vorzugsweise THF, Dichlormethan, Aceton, Acetonitril, Dimethoxyethan und Dioxan. Besonders erwähnt sei Acetonitril. Die Diazotierung erfolgt im allgemeinen zwischen -20 und +30°C innerhalb von 1 bis 48 h.

Bei der erfindungsgemäßen Herstellung von Verbindungen der allgemeinen Formel (2) durch die Verknüpfung von Verbindungen der allgemeinen Formeln (4) und (5), in welcher X und R² die angegebene Bedeutung haben und Y für Diazo (N₂}--steht, können die beiden Reaktionspartner in äquivalenten Mengen eingesetzt werden. Es kann jedoch auch ein Partner - am günstigsten die Verbindung der allgemeinen Formel (5) im Überschuß verwendet werden. In bestimmten Fällen kann die Verbindung der allgemeinen Formel (5) sogar als Lösungsmittel dienen. Als saure Katalysatoren kommen Protonensäuren, beispielsweise Trifluoressigsäure, Trifluormethansulfonsäure, Perchlorsäure, Tetrafluorborwasserstoffsäure, Chlorwasserstoffsäure, Quadratsäure, p-Toluolsulfonsäure, Campfersulfonsäure, Polyphosphorsäure oder Lewissäuren, beispielsweise Bortrifluorid, Zink(II)chlorid, Aluminiumchlorid, Zinn(IV)chlorid, Quecksilber(II)chlorid, Siliciumtetrachlorid, Zinn(II)chlorid, Titan(IV)chlorid, Antimon(V) chlorid, Eisen(III)chlorid, Antimon(III)chlorid, und außerdem Trimethylsilyltrifluormethansulfonat und Trimethylsilyltrifluoracetat sowie saure Ionenaustauscher und Kieselgel in Betracht. Im allgemeinen können bis zu molaren Mengen des sauren Katalysators zugesetzt werden. Bevorzugt wird jedoch in Gegenwart katalytischer Mengen (d.h. 1 - 25 mol-%) gearbeitet.

Als Verdünnungsmittel kommen alle inerten Lösungsmittel in Betracht. Hierzu gehören beispielsweise Dimethoxyethan, Diglyme, Triglyme, Tetrahydrofuran, Dioxan, Diethylether, t-Butylmethylether, Dichlormethan, Tetrachlorkohlenstoff, 1,2-Dichlorethan, 1,1,2-Trichlorethan, Trichlorethylen, Chlorbenzol, Dichlorbenzol, Chloroform, Ethylacetat, Toluol, Cyclohexan, Acetonitril und Mitromethan. Die Umsetzung erfolgt im allgemeinen zwischen -30 und +50°C, vorzugsweise jedoch bei Raumtemperatur innerhalb von 0,1 bis 10 h. Die Umsetzungsgeschwindigkeit ist von der Menge des verwendeten Katalysators abhängig.

Bei der erfindungsgemäßen Herstellung von Verbindungen der allgemeinen Formel (3) durch eine Verknüpfung von Verbindungen der allgemeinen Formeln (4) und (5), in welcher X und R² die angegebene Bedeutung haben und Y für Wasserstoff (H₂) steht, können die beiden Reaktionspartner in äquivalenten Mengen eingesetzt werden. Es kann jedoch auch ein Partner - am günstigsten die Verbindung der allgemeinen Formel (5) im Überschuß verwendet werden. In bestimmten Fällen kann die Verbindung der allgemeinen Formel (5) sogar als Lösungsmittel dienen. Als saure Katalysatoren kommen Protonensäuren, beispielsweise Trifluoressigsäure, Trifluormethansulfonsäure, Perchlorsäure, Tetrafluorborwasserstoffsäure, Chlorwasserstoffsäure, Quadratsäure, p-Toluolsulfonsäure, Campfersulfonsäure, Polyphosphorsäure oder Lewissäuren, beispielsweise Bortrifluorid, Zink(II)chlorid, Aluminiumchlorid, Zinn(IV) chlorid, Quecksilber(II)chlorid, Siliciumtetrachlorid, Zinn(II)chlorid, Titan(IV)chlorid, Antimon(V)chlorid, Eisen(III)chlorid, Antimon(III)chlorid, und außerdem Trimethylsilyltrifluormethansulfonat und Trimethylsilyltrifluoracetat sowie saure Ionenaustauscher und Kieselgel in Betracht. Im allgemeinen können bis zu molaren Mengen des sauren Katalysators zugesetzt werden. Bevorzugt wird jedoch in Gegenwart katalytischer Mengen (d.h. 1-10 mol-%) gearbeitet.

Als Verdünnungsmittel kommen alle inerten Lösungsmittel in Betracht. Hierzu gehören beispielsweise Dimethoxyethan, Diglyme, Triglyme, Tetrahydrofuran, Dioxan, Diethylether, t-Butylmethylether, Dichlormethan, Tetrachlorkohlenstoff, 1,2-Dichlorethan, 1,1,2-Trichlorethan, Trichlorethylen, Chlorbenzol, Dichlorbenzol, Chloroform, Ethylacetat, Toluol, Cyclohexan, Acetonitril und Nitromethan. Die Umsetzung erfolgt im allgemeinen zwischen -30 und +50°C, vorzugsweise jedoch bei Raumtemperatur innerhalb von 0,1 bis 10 h. Die Umsetzungsgeschwindigkeit ist von der Menge des verwendeten Katalysators abhängig.

Die erfindungsgemäße Herstellung von Verbindungen der allgemeinen Formel (3) durch Umsetzung von Verbindungen der allgemeinen Formel (4) mit Verbindungen der allgemeinen Formel (6) erfolgt unter denselben Bedingungen, wie sie oben für die Umsetzung von Verbindungen der allgemeinen Formel (4) mit Verbindungen der allgemeinen Formel (5) beschrieben ist. Die Dreifachbindung der dabei entstehenden Zwischenverbindung muß im Anschluß noch hydratisiert werden. Zur Hydratisierung der Dreifachbindung können literaturbekannte Verfahren verwendet werden, wie sie beispielsweise von S.Ikegami et al., Tetrahedron Lett. 2875 (1982) beschrieben wurden. Dieses Vorgehen wird weiterhin in den Beispielen eingehend illustriert.

Die folgenden Beispiele dienen der weiteren Beschreibung der Erfindung, ohne diese zu beschränken.

### Beispiel 1

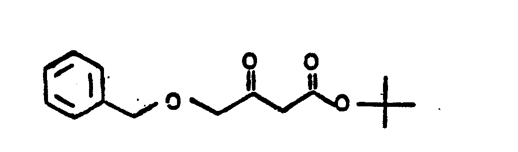

### 4-Benzyloxyacetessigsäure-tert.-butylester

Zu einer eisgekühlten Suspension von 18,0 g (0,6 mol) Natriumhydrid (80 % in Paraffinöl)in 150 ml wasserfreiem THF wurde unter Eiskühlung eine Lösung von 64,9 g 62.1ml(0,6 mol) Benzylalkohol in 50 ml THF innerhalb von 1 h getropft. Danach wurde 0,5 h bei Raumtemp. nachgerührt. Dazu tropfte man innerhalb von 1 h bei 0°C eine Lösung von 57,8 g (0,3 mol) 4-Chloracetessigsäure- tert.-butylester in 50 ml THF. Das Eisbad wurde entfernt, man rührte noch 1 h bei Raumtemperatur, und neutralisierte die Reaktionsmischung durch vorsichtige Zugabe von 0,5 N HC1 unter Kühlung (pH-Kontrolle).

Man extrahierte mehrmals mit Ether, wusch die vereinigten Etherextrakte mit Wasser und trocknete über MgS0₄. Abdampfen des Ethers i.Vak. ergab ein öl, welches durch Chromatographie an 1,7 kg Kieselgel (Toluol:Ethylacetat 95:5) gereinigt wurde. Man erhielt 41,7 g (53 %) 4-Benzyloxyacetessigsäure-tert.-butylester, R_{F}: 0,47 (Toluol:Ethylacetat 9:1). ¹H-NMR (200 MHz, CDCl₃) δ1,50 (s, 9H, C(CH₃)₃), 3,48 (s, 2H, CH₂), 4,18 (s, 2H, CH₂), 4,63 (s, 2H, CH₂), 7,40 (s, SH,Ph). IR (CHCl₃) 1740-1710 (C=O, ß-Ketoester), 1656 cm⁻¹ (C=C, Enolform). C₁₅H₂₀O₄ (²64.3) Ber.: 68,2 H 7,6 Gef.: 68,2 7,6

### Beispiel 2

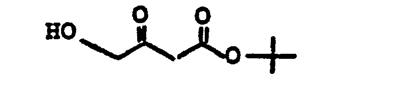

### 4-Hydroxyacetessigsäure-tert.-butylester

Eine Mischung von 963 mg (5 mmol) 4-Benzyloxyacetessig- säure-tert.-butylester und 200 mg Palladium (10 % auf Kohle) in 50 ml Methanol wurde unter einer Wasserstoffatomosphäre (1 at) 1,5 h bei Raumtemperatur gerührt. Der Katalysator wurde abfiltriert und mit etwas Dichlormethan gewaschen. Die Filtratlösung wurde i.Vak. abgedampft und der Rückstand durch 3 g neutrales Aluminiumoxid filtriert (Dichlormethan). Das Lösungsmittel wurde i.Vak. abgedampft und das zurückbleibende öl am Hochvak. getrocknet. Man erhielt 567 mg (65 %)4-Hydroxyacetessigsäu- re-tert.-butylester, welcher sofort für weitere Umsetzungen verwendet wurde.

R_{F}: 0,31 (Toluol:Ethylacetat 3:2) ¹H-NMR (200 MHz, CDC1₃) 1,50 (s, 9H, C(CH₃)₃), 3,05 (bs, 1H, OH), 3,43 (s, 2H, CH₂), 4,38 (bs, 2H, CH2). C₈H₁₄O₄ (174.2) Ber.: C 55,16 H 8,10 Gef.: 55,2 8,3

### Beispiel 3

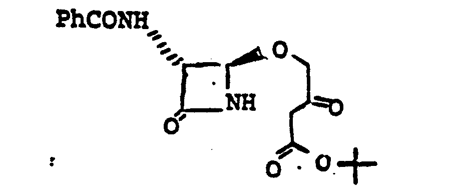

### 4-[3(R)-Benzoylamino-2-azetidinon-4(R)-yloxy]acetessig- säure-tert.-butylester

Eine Suspension von 216 mg (1,15 mmol) (1R, 5S)-3-Phenyl-4-oxa-2,6-diazabicyclo[3.2.0]hept-2-en-7-on und 400 mg (2,3 mmol) frisch hergestelltem 4-Hydroxyacetessigsäure- tert.-butylester in 4 ml wasserfreiem THF wurde bei Raumtemperatur mit 30 µl Bortrifuoridetherat versetzt. Nach kurzer Zeit entstand eine klare, hellgelbe Lösung, welche 0,5 h bei Raumtemperatur gerührt wurde. Danach wurde in verdünnte NaHCO₃ 3-Lösung gegossen. Man extrahierte mit Dichlormethan, wusch mit Wasser und trocknete über Magnesiumsulfat. Nach Abdampfen des Lösungsmittels i.Vak. und Chromatographie des Rückstandes an 60 g Kieselgel (Toluol:Ethylacetat 1:3) erhielt man 128 mg (31 %) 4-[3(R)-Benzoylamino-2-azetidinon-4(R)-yloxy]-acetessigsäure-tert.-butylester als farblose Kristalle, Schmp.: 142°C, R_{F}: 0,36 (Toluol:Ethylacetat 1:4), [α]²⁰_{D} = -32.24° (0.955 proz. in CHCl₃).

IR (KBr) : 3320 (NH), 1763 (C=_{O}, ß-Lactam), 1738 (C=O, Keton), 1720 (C=O, Ester), 1656 und 1524 cm⁻¹ (Amid).

¹H-NMR (200 MHz, CDC1₃) δ1,45 (s, 9H, C(CH₃)₃), 3,43 (s, 2H, COCH₂COOR), 4,50 (AB, J=15 Hz, 2H, OCH₂CO), 4,78 (dd, J=1 Hz, 8 Hz, 1H, H-3), 5,15 (d, J=1 Hz, 1H, H-4), 7,13 (s, 1H, NH), 7,3-7,6 (m, 4H, Ph, NH), 7,8 (m, 2H, ortho-Benzoyl-H).

C₁₈H₂₂N₂0₆ (362.4) Ber.: C 59,66 H 6,12 N 7,73 Gef.: 59,5 5,2 7,6

### Beispiel 4

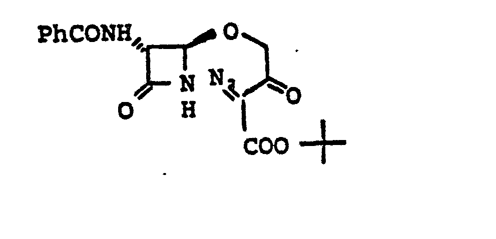

### 4-[3(R)-Benzoylamino-2-azetidinon-4(R)-yloxy]-2-diazo-3-oxo-buttersäure-tert.-butylester

Eine auf O°C gekühlte Lösung von 184 mg (0,5 mmol) 4-[3(R)-Benzoylamino-2-azetidinon-4(R)- yloxy]acetessig- säure-tert.-butylester und 100 mg (0,55 mmol) frisch hergestelltem p-Toluolsulfonsäureazid in 10 ml wasserfreiem Acetonitril wurde tropfenweise mit 258 µl (1,85 mmol - 3,75 equiv.) Triethylamin versetzt. Das Kühlbad wurde entfernt und man rührte 1,5 h bei Raumtemp. Danach wurde das Reaktionsgemisch i.Vak. eingeengt und an 8 g Kieselgel (Toluol:Ethylacetat 35:65) chromatographiert. Man erhielt 186 mg (96 %) 4-[3(R)-Benzoyl- amino-2-azetidinon-4(R)-yloxy]-2-diazo-3-oxo-buttersäure- tert.-butylester als helle Kristalle, Schmp.: 127°C (Zers.), R_{F}: 0,30 (Toluol:Ethylacetat 35:65), [α]²⁰_{D} -15,4° (0,867 proz. in CHCl₃).

IR (KBr): 3307 (NH); 2154 (N₂), 1786 (C=O, ß-Lactam), 1699 (C=O, Ester), 1643 (Amid I), 1527 cm⁻¹ (Amid _{II}).

¹H-NMR (250 MHz, CDCl₃)δ1,54 (s, 9H, C(CH₃)₃), 4,77 (dd, J=7 Hz, ~1Hz, 1H, H-3), 4,89 (AB, J=17 Hz, 2H, CH₂0), 5,26 (d, J= ~ 1 Hz, 1H, H-4), 7,1-7,5 (m, 5H, NH, NH, Ph), 7,8 (m, 2H, o-Benzoyl-H).

C₁₈H₂₀N₄O₆ (³⁸⁸.4) Ber.: C 55,67 H 5,19 N 14,43 Gef.: 55,7 5,4 13,9

### Beispiel 5

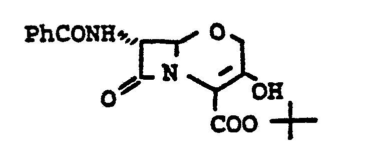

### (6R, 7R)-7-Benzoylamino-3-hydroxy-8-oxo-5-oxa-1-azabi- cyclo[4.2.0]oct-2-en-2-carbonsäure-tert.-butylester

a) Eine Suspension von 341 mg (0,88 mmol) 4-L3(R)-Ben- zoylamino-2-azetidinon-4(R)-yloxy]-2-diazo-3-oxo- buttersäure-tert.-butylester und 0,5 mg Rhodium(II)-acetat in 18 ml wasserfreiem Benzol wurde für 10 min. durch Einleiten von trockenem Stickstoff sauerstofffrei gemacht.

Die Reaktionsmischung wurde dann 1 h auf 80°C erhitzt, wobei das feste Ausgangsmaterial allmählich in Lösung ging. Die Mischung durfte abkühlen, der Katalysator wurde durch Filtration (Kieselgur) abgetrennt und mit Dichlormethan gewaschen.

Das Lösungsmittel wurde i.Vak. abgedampft und der Rückstand an 20 g Kieselgel (Toluol:Ethylacetat 35:65) chromatographiert. Man erhielt 260 mg (82 %) (6R, 7R)-7-Benzoylamino-3-hydroxy-8-oxo-5-oxa-1-azabicyclo[4.2.0]oct-2-en-2-carbonsäure-tert.-butylester als farblosen Hartschaum, R_{F}: 0,36 (Toluol:Ethylacetat 3:7).

IR (KBr) : 3324 (OH, NH), 1771 (C=O, ß-Lactam), 1655 (ß-Ketoester, Enol-form), 1655 und 1532 cm⁻¹ (Amid).

¹_{H}-_{NMR} (250 _{MH}z, CDCl₃)δ1,59 (s, 9H, C(CH₃)₃), 4,42 (AB, J= 17,5 Hz, 2H, CH₂0), 4,93 (d, J= 7,5 Hz, 1H, H-7), 5,05 (s, 1H, H-6), 7,05 (d, J=7,5 Hz, 1H, NH) 7,3-7,6 (m, 3H, Ph) 7,8 (m, 2H, ortho-Benzoyl-H).

C₁₈H₂₁N₂O₆ (361.4) Ber.: C 59,83 H 5,86 N 7,75 Gef.: 59,8 5,6 7,9

b) Eine wasserfreie und entgaste Lösung von 78 mg (0,2 mmol) 4-L3(R)-Benzoylamino-2-azetidinon-4-(R)-yloxy7-2-diazo-3-oxo-buttersäure-tert.-butylester in 25 ml Benzol:THF 3:1 wurde bei 20°C für 1 h mit einer 450 W Quecksilberdampflampe mit Pyrex-Filter in einer Stickstoffatomosphäre bestrahlt. Das Lösungsmittel wurde i.Vak. abgedampft und der Rückstand durch Chromatographie an 8 g Kieselgel (Toluol:Ethylacetat 35:65) gereinigt. Man erhielt 12 mg (17 %) der Titelverbindung als farblosen Hartschaum.

Die physikalischen Daten waren identisch mit der nach Methode a) hergestellten Verbindung.

### Beispiel 6

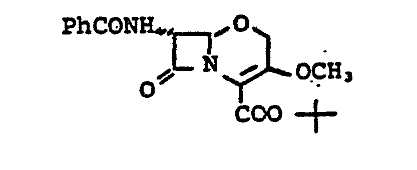

### (6R, 7R)-7-Benzoylamino-3-methoxy-8-oxo-5-oxa-1-aza- bicyclo[4.2.0]oct-2-en-2-carbonsäure-tert.-butylester

Eine Lösung von 260 mg (0,72 mmol) (6R, 7R)-7-Benzoyl- amino-3-hydroxy-8-oxo-5-oxa-1-azabicyclol-[4.2.0]oct-2-en-2-carbonsäure-tert.-butylester in 3 ml wasserfreiem Dichlormethan wurde bei Raumtemperatur tropfenweise mit einer Lösung von Diazomethan in Ether versetzt bis eine gelbe Färbung erhalten blieb. Anschließend wurden das Lösungsmittel und überschüssiges Diazomethan i.Vak. abgedampft und der Rückstand an 18 g Kieselgel (Toluol: Ethylacetat 2:3) chromatographiert. Man erhielt 169 mg (62 %) der Titelverbindung als farblosen Hartschaum. R_{f}: 0,53 (Toluol:Ethylacetat 1:4).

IR (KBr): 3354 (NH), 1775 (C=O, B-Lactam), 1722 (C=O, Ester),1650 und 1531 cm⁻¹ (Amid).

¹H-NMR (250 MHz, CDCl₃)δ1,53 (s, 9H, C(CH₃)₃), 3,78 (s, 3H, OCH₃), 3,36, 4,48 (d, J=18 Hz, 2H, CH₂0), 5,00 (s, 1H, H-6), 5,04 (d, J=7,5 Hz, 1H, H-7), 7,3-7,6 (m, 4H, Ph, NH) 7,85 (m, 2H, o-Benzoyl-H).

C₁₉H₂₂N₂O₆ (^{374.}4) Ber.: C 60,95 H 5,92 _{N} 7,₄₈ Gef.: 60,9 5,8 7,3

### Beispiel 7

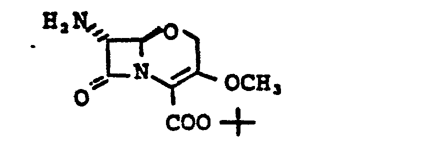

### (6R, 7R)-7-Amino-3-methoxy-8-oxo-5-oxa-1-azabicyclo [4.2.0]oct-2-en-2-carbonsäure-tert.-butylester

Zu einer auf -20°C gekühlten Lösung von 749 mg (2 mmol) (6R, 7R)-7-Benzoylamino-3-methoxy-8-oxo-5-oxa-1-azabicyclo-[4.2.0]oct-2-en-carbonsäure-tert.-butylester in 3,7 ml wasserfreiem Dichlormethan gab man 0,4 ml (5 mmol -2,5 equiv.) Pyridin und dann auf einmal 0,83 g (4 mmol - 2 equiv.) Phosphorpentachlorid. Man rührte 10 min. bei 0°C und 1 h bei + 15°C, kühlte auf -60°C und gab rasch 12,2 ml vorgekühltes wasserfreies Methanol zu und rührte 30 min. bei Raumtemperatur nach. Dann wurde auf -15°C gekühlt und man tropfte 1.6 ml destilliertes Diethylamin zu, rührte noch 10 Minuten bei ca. -10°C und goß zur Aufarbeitung in eiskalte gesättigte NaNCO₃-Lösung. Man extrahierte mehrmals mit Dichlormethan, wusch mit Wasser und trocknete über MgS0₄. Das Lösungsmittel wurde i. Vak. abgedampft und der Rückstand an 60 g Kieselgel (Ethylacetat:Aceton 85:15) chromatographiert. Man erhielt 158 mg (29 %) der Titelverbindung als hellen Hartschaum, R_{F}: 0,33 (Ethylacetat:Aceton 85:15).

IR (KBr): 1771 (C=O, B-Lactam), 1720 (C=O, Ester), ₁₆₀₃ Cm⁻¹ (O-C=C).

¹H-NMR (250 MHz, CDCl₃)δ1,5 (s, 9H, C(CH₃)₃), 1,75 (bs, 2H, NH₂), 3,77 (s, 3H, OCH₃), 4,06 (s, 1H, H-7), 4,45 (AB, J=17 Hz, 2H, CH₂0), 4,79 (s, 1H, H-6).

### Beispiel 8

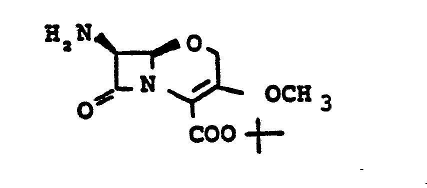

### (6R, 7S)-7-Amino-3-methoxy-8-oxo-5-oxa-1-azabicyclo [4.2.0]oct-2-en-2-carbonsäure-tert.-butylester

Zu einer Lösung von 1,35 g (5 mmol) (6R, 7R)-7-Amino-3-methoxy-8-oxo-5-oxa-1-azabicyclo[4.2.0]oct-2-en-2-carbonsäure-tert.-butylester in 81 ml Benzol gab man 7,7 g Molekularsieb 3Å und nach einigen Minuten 2,3 ml (23,5 mmol -4,7 equiv.) wasserfreien Trichloracetaldehyd. Die Mischung wurde unter Rühren 2,5 h zum Sieden erhitzt, abgekühlt, rasch filtriert und i.Vak. eingeengt. Der Rückstand wurde in 23 ml wasserfreien THF gelöst und auf -40°C gekühlt. Man gab 0,89 ml (5,1 mmol - 1.02 equiv.) Ethyldiisopropylamin zu, rührte 20 min. bei -40°C und ließ dann auf 0°C erwärmen. Bei dieser Temperatur wurde rasch eine vorgekühlte Lösung von 2,18 mg Kaliumborhydrid in 35 ml 50 proz. wäßr. THF zugegeben und es wurde 5 min. gerührt. Anschließend wurde bei 0°C mit einer Mischung aus 38 ml 2N HCl und 17 ml Acetonitril gequencht und noch 2 h nachgerührt. Zur Aufarbeitung goß man in gesättigte NaHCO₃-Lösung, extrahierte mit Dichlormethan, wusch mit Wasser und trocknete über MgS0₄. Nach Abdampfen des Lösungsmittels i.Vak. und Chromatographie des Rückstands an 120 g Kieselgel (Ethylacetat:Aceton 6:4) erhielt man 372 mg (28 %) der Titelverbindung als farblosen Hartschaum.

IR (KBr) : 1773 (C=O, ß-Lactam), 1718 (C=O, Ester), ₁₆₁₁ cm⁻¹ (O-C=C).

¹H-NMR (250 MHz, CDCl₃)δ1,5 (s, 9H, C(CH3)3), 1,68 (bs, 2H, NH₂), 3,75 (s, 3H, OCH₃), 4,44, 4,56 (AB, J=₁₇,5 Hz, CH₂O), 4,45 (d, J=4,5 Hz, H-7) Zus. 3H, 4,97 (d, J=4,5 Hz, 1H, H-6).

### Beispiel 9

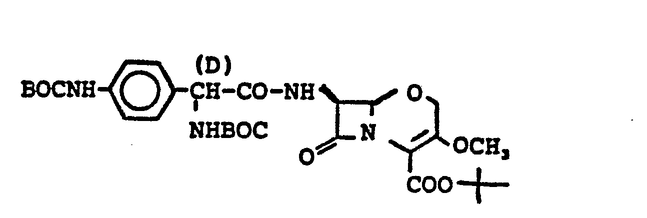

### (6R, 7S)-7-[(2R)-1-tert.-Butoxycarbonylamino-2-(4-tert.-butoxycarbonylaminophenyl)acetamido7-3-methoxy-8-oxo-5-oxa-1-azabicyclo[4.2.0]oct-2-en-2-carbonsäure-tert.-butylester

Zu einer auf -50°C gekühlten Lösung von 495 mg (1,35 mmol) N,N'-Bis-(tert.-butoxycarbonyl)-4-amino-D-phenylglycin ([α]²⁰_{D} = -106.5°, 1.033 proz. in Methanol) in 8 ml wasserfreiem Dichlormethan wurden nacheinander 235 µl (1,35 mmol) Ethyldiisopropylamin und 105 µl (1,35 mmol) Methansulfonylchlorid getropft. Man rührte 45 min. bei -50°C und tropfte dann eine Lösung von 354 mg (1,31 mmol) (6R, 7S)-7-Amino-3-methoxy-8-oxo-5-oxa-1-azabicyclo-[4.2.0]oct-2-en-2-carbonsäure-tert.-butylester und 235 µl (1,35 mmol) Ethyldiisopropylamin in 6,5 ml wasserfreiem Dichlormethan zu. Es wurde noch 15 min bei -50°C gerührt, dann durfte sich die Mischung auf -10°C erwärmen. Zur Aufarbeitung wurde in eine Mischung aus 100 ml Eiswasser und 50 ml Dichlormethan gegossen, mit Dichlormethan extrahiert, mit NaHC0₃-Lösung und Wasser gewaschen und über MgSO₄ getrocknet. Nach Abdampfen des Lösungsmittels und Chromatographie des Rückstands an 45 g Kieselgel (Toluol:Ethylacetat 1:1) erhielt man 422 mg (52 %) der Titelverbindung als farblosen Hartschaum.

IR(KBr) : 1783 (C=O, B-Lactam), 1725 (C=O, Ester), 1680 und 1520 cm⁻¹ (Amid).

¹H-NMR (250 MHz, CDCl₃)δ1,42, 1,50, 152, 1,60 (s, 27H, C(CH₃)₃), 3,76 (s, 3H, OCH₃), 4,34, 4,43 (AB, J=16 Hz, 2H, CH₂0), 5,0 (d, J=4 Hz, 1H, H-6), 5,17 (bs, 1H, CH), 5,6 (dd, J= 4 Hz, 9,5 Hz, 2H, H-7, NH), 6,55 (bs, 2H, NH), 7,3, 7,5 (d, J= 8,5 Hz, ^{4H,} H_{arom.}).

### Beispiel 10

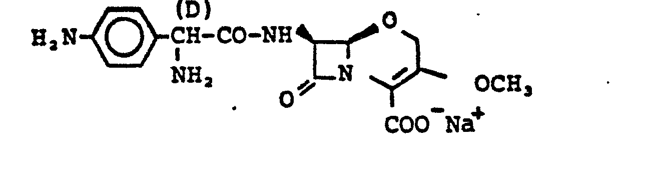

### Natrium-(6R, 7S)-7-[(2R)-2-Amino-2-(4-aminophenyl)acet- amido]-3-methoxy-8-oxo-5-oxa-1-azabicyclo[4.2.0]oct-2-en-2-carboxylat

Eine Lösung von 414 mg (0,67 mmol) (6R, 7S)-7-[(2R)-2-tert.-Butoxycarbonylamino-2-(4-tert.-butoxycarbonyl- aminophenyl)-acetamido]-3-methoxy-8-oxo-5-oxa-1-aza- bicyclo[4.2.0]oct-2-en-2-carbonsäure-tert.-butylester in 10 ml wasserfreier Trifluoressigsäure wurde 1 h bei Raumtemperatur gerührt. Danach wurde 15 ml Benzol zugegeben und die Mischung wurde i.Vak. abgedampft. Der Rückstand wurde mit Ether verrieben und das dabei entstehende Pulver wurde abgesaugt, mit Ether gewaschen und in 20 ml bidestilliertem Wasser gelöst. Die Wasserphase wurde mehrmals mit Ether extrahiert, i.Vak. von Etherresten befreit und durch eine Säule mit Amberlite IRA-68 (Acetat-Form) filtriert und mit 200 ml Wasser eluiert. Nach Gefriertrocknen des Eluats erhielt man 166 mg (65 %) der Titelverbindung in einer Reinheit von 96 % (HPLC).

IR (KBr) : 3500-2900 (b), 1764 cm⁻¹ (C=O, ß-Lactam).

¹H-NMR (250 MHz, DMSO)δ3,74 (s, 3H, OCH₃), 4,39, 4,46 (AB, J=17 Hz, CH₂0), 4,2-4,6 (m, NH₂, CH) Zus. 7H, 5,05 (d, J= 4 Hz, 1H, H-6), 5,43 (dd, J= 4 Hz, 9 Hz, 1H, H-7), 6,54, ₇,_{09 (d}, _{J= 10 Hz}, 4H, H_{arom.}), 8,82 (d, J= 9 Hz, 1H, NH).

### Beispiel 11

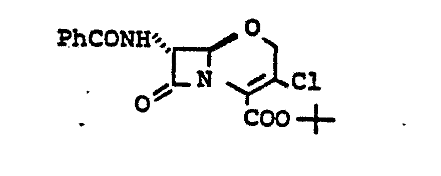

### (6R, 7R)-7-Benzoylamino-3-chloro-8-oxo-5-oxa-1-azabi- cyclo(4.2.0]oct-2-en-2-carbonsäure-tert.-butylester

Zu einer auf 0°C gekühlten Lösung von 7,82 g (21,7 mmol) (6R, 7R)-7-Benzoylamino-3-hydroxy-8-oxo-5-oxa-1-azabicyclo [4.2.0]oct-2-en-2-carbonsäure-tert.-butylester in 430 ml wasserfreiem DMF wurden 30,5 ml (32,6 mmol -1,5 equiv.) Phosphortrichlorid getropft. Das Kühlbad wurde entfernt und man rührte 2 h bei Raumtemp.. Zur Aufarbeitung wurde in ein Gemisch aus Dichlormethan und Eiswasser gegossen, mit Dichlormethan extrahiert (2x), mit NaHCO₃-Lösung und Wasser (2x) gewaschen und über MgSO₄ getrocknet. Nach Abdampfen des Lösungsmittels i.Vak. und Chromatographie des Rückstands an 340 g Kieselgel (Toluol:Ethylacetat 7:3) erhielt man 2,47 g (30 %) der Titelverbindung als farblosen Hartschaum.

IR (KBr): 3360 (NH), 1787 (C=O, B-Zactam), 1721 (C=O, Ester), 1650 und 1518 cm⁻¹ (Amid).

¹H-NMR (250 MHz, CDCk₃) δ 1,55 (s, 9H, C(CH₃)₃), 4,35-4,48 (AB, J=17,5 Hz, 2H, CH₂0), 4,92 (s, 1H, H-6), 5,18 (d, J=7,5 Hz, 1H, H-7), 7,3-7,6 (m, 4H, Ph, NH), 7,9 (m, 2H, o-Benzoyl-H).

### Beispiel 12

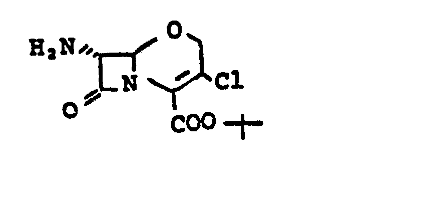

(6R, 7R)-7-Amino-9-chloro-8-oxo-5-oxa-1-azabicyclo[4.2.0] oct-2-en-2-carbonsäure-tert.-butylester

Wie für Beispiel 7 beschrieben erhielt man aus 1,19 g (3,15 mmoll (6R, 7R)-7-Benzoylamino-3-chloro-8-oxo-5-oxa-1-aza-bicycolo[4.2.0]oct-2-en-2-carbonsäure-tert.-butylester nach Chromatographie des Rohprodukts an 93 g Kieselgel (Toluol:Ethylacetet 1:4) 308 mg (36 %) der Titelverbindung als helles Pulver.

IR (KBr): 3387 (NH₂ as.), 3320 (NH₂ sym), 1780 (C=O, B-Lactam), 1726 (C=_{O}, Ester, 1609 cm⁻¹ (_{C}=_{C}).

¹H-NMR (250 MHz, CDC1₃) δ 1,53 (s, 9H, C(CH₃)₃), 1,9 (bs, 2H, NH₂) , 4,13 (s, 1H, H-7), 4,4, 4,48, (AB, H=17 Hz, 2H, CH₂0), 4,86 (s, 1H, H-6).

### Beispiel 13

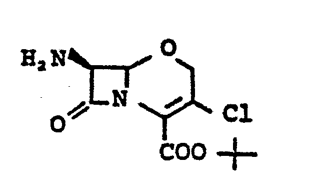

### (6R, 7S)-7-Amino-3-chloro-8-oxo-5-oxa-1-azabicyclo[4.2.0] oct-2-en-2-carbonsäure-tert.-butylester

Wie für Beispiel 8 beschrieben erhielt man aus 2,39 g (8,7 mmol) 6R, 7R)-7-Amino-3-chloro-8-oxo-5-oxa-1-azabi- cyclo[4.2.0]oct-2-en-2-carbonsäure-tert.-butylester und Chromatographie des Rohprodukts an 80 g Kieselgel (Ethylacetat) 837 mg (35 %) der Titelverbindung als hellen Hartschaum.

IR (KBr): 3399 (NH₂as.), 3320 (NH₂ sym.), 1787 (C=O, B-Lactam) , 1728 (C=O, Ester), 1606 cm⁻¹ (_{C}=_{C}).

¹H-NMR (250 MHz, CDCl₃)δ 1,5 (s, C(CH₃)₃), 1,75 (bs, NH₂), Zus. 11H, 4,48, 4,54 (AB, J= 16 Hz, 2H, CH₂0), 4,59 (d, J= 4,5 Hz, 1H, H-7), 5,1 (d, J= 4,5 Hz, 1H, H-6).

### Beispiel 14

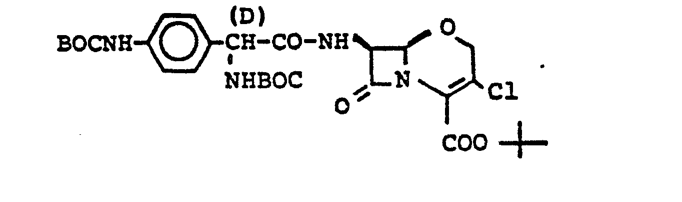

(6R, 7S)-7-[(2R)-2-tert.-Butoxycarbonylamino-2-(4-tert.-butoxycarbonylaminophenyl)acetamido]-3-chloro=8-oxo-5-oxa-1-azabicyclo[4.2.0]oct-2-en-2-carbonsäure-tert.-butylester

Wie für Beispiel 9 beschrieben erhielt man aus 934 mg (3,4 mmol) (6R, 7S)-7-Amino-3-chloro-8-oxo-5-oxa-1-aza- bicyclo[4.2.0]oct-2-en-2-carbonsäure-tert.-butylester und 1,28 g (3,5 mmol) N,N'-Bis(tert.-butoxycarbonyl)-4-amino- _{D}-phenylglycin nach Chromatographie an 155 g Kieselgel (Toluol:Ethylacetat 7:3) 805 mg (38 %) der Titelverbindung als farblosen Hartschaum.

IR (KBr): 1803 (C=O, B-Lactam), 1730 (C=O, Ester), 1680 und 1520 cm⁻¹ (Amid).

¹_{H}-_{NMR} (_{250 MH}z, CDCl₃)δ 1,42, 1,50, 1,53, 1,5₈ (s, 27H, C(CH₃)₃), 4,4 (AB, J= 17 Hz, 2H, CH₂0), 5,1 (d, J=4 Hz, H-6), 5,15 (bs, CH), Zus. 2H, 5,5 (bs, 1H, NH), 5,7 (dd, J=₄ Hz, 10Hz, 1H, H-7), 6,52, 6,54 (bs, 2H, NH), 7,3, 7,5 (d, J=9 Hz, 4H, H_{arom.}).

### Beispiel 15 -

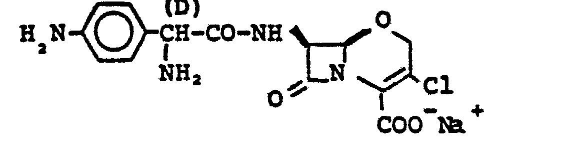

### Natrium-(6R, 7S)-7-[(2R)-2-Amino-2-(4-aminophenyl)-acet- amido]-3-chloro-8-oxo-5-oxa-1-äzabicyclo-[4.2.0]oct-2-en-2-carboxylat

Wie für Beispiel 10 beschrieben erhielt man aus 760 mg (1,22 mmol) (6R, 7S)-7-[(2R)-2-tert.-Butoxycarbonylamino-2-(4-tert.-butoxycarbonylaminophenyl)acetamido]-3-chloro-8-oxo-5-oxa-1-azabicyclo[4.2.0]oct-2-en-2-carbonsäure- tert.-butylester 361 mg (76 %) der Titelverbindung in einer Reinheit von 89 % (HPLC).

IR (KBr): 3500-2900 (b), 1776 (C=O, B-Lactam), 1680 und 1517 (Amid), 1609 cm⁻¹ (COONa).

¹H-NMR (250 MHz, DMSO) δ 3,5-4,5 (b, NH₂), 4,1, 4,37 (AB, J=17 Hz, CH₂O) , 4,8 (m, CH), Zus. 7H, 5,11 (d, J=4 Hz, 1H, H-6), 5,49 (dd, J=4 Hz, 9 Hz, 1H, H-7), 6,54, 7,11 (d, J= 9,5 ^{Hz, 4H,} Hₐᵣₒₘ.) , 9,14 (d, J₌ 9 Hz, 1H, NH).

### Beispiel 16

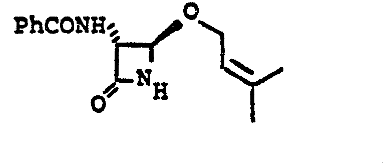

### 3(R)-Benzoylamino-4-(Ry-[3-Methvl-2-butenyloxy]-2-azetidinon

Zu einer Suspension von 150 mg (0,78 mmol) (1R, 5S)-3-phenyl-4-oxa-2,6-diazabicyclo[3.2.0]hept-2-en-7-on in 1,3 ml wasserfreiem Dichlormethan und 0,81 ml (7,8 mmol - 10 equiv.) 3-Methyl-2-buten-1-ol gab man bei 0°C auf einmal 181 mg (0,96 mmol - 1,2 equiv.) wasserfreies Zinn(II)-chlorid (getrocknet durch kurzes Schmelzen im Hochvak.) Das Kühlbad wurde entfernt und nach einigen Minuten entstand eine klare Lösung. Man rührte noch 15 min bei Raumtempertur, goß die Reaktionsmischung in verd. NaHCO₃-LÖsung, extrahierte mit Dichlormethan, wusch mit Wasser und trocknete über MgSO₄. Nach Abdampfen des Lösungsmittels i.Vak. und Filtration des Rückstands an 5 g Kieselgel (Toluol:Ethylactat 3:7) erhielt man 153 mg (70 %) der Titelverbindung als farblose Kristalle, Schmp.: 92°C, R_{F}: 0,38 (Toluol:Ethylacetat 3:7).

IR (KBr) : 1775 (C=O, B-Lactam), 1667 (C=O, Amid), 1633 (_{C}=_{C}), 15₂9 cm⁻¹ (Amid _{II}-Bande).

¹H-MNR (200 MHz, CDCl₃)δ 1,70, 1,77 (s, 6H, CH₃), 4,18 (m, 2H, -OCH₂-CH=), 4,74 (dd, J= 9 Hz, 1 Hz, 1H, H-3), 5,23 (d, J= 1 Hz, 1H, H-4), 5,38 (m, 1H, -CH=), 6,78 (s, 1H, NH), 7,18 (d, J= 9,Hz, 1H, NH), 7,4-7,6 (m, 3H, C₆H₅), 7,8 (m, 2H, o-C₆H₅).

MS (70eV): m/e = 274 (M⁺) ; ber.: 274,32

C₁₅H₁₈N₂0₃ (274.3) Ber.: C 65,7 H 6,6 N 10,2 Gef.: 65,4 6,5 10,0.

### Beispiel 17

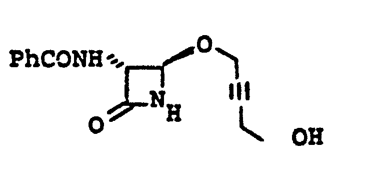

### 4-[3(R)-Benzoylam3.no-2-azetidinon-4-(R)-yloxy]-2-butin-1-ol

Eine Suspension von 941 mg (5 mmol) (1R, 5S)-3-Phenyl-4-oxa-2,6-diazabicyclo[3.2.0]hept-2-en-7-on und 861 mg (10 mmol - 2 equiv.) 2-Butin-1,4-diol in 20 ml wasserfreiem THF wurde bei Raumtemperatur mit 100 µl Bortrifluoridetherat versetzt. Man rührte 2 h bei Raumtemperatur wobei zunächst eine klare Lösung entstand, aus welcher sich später ein fester Niederschlag abschied. Der Niederschlag wurde abgesaugt und die Filtratlösung in verd. NaHC0₃-Lösung gegossen. Man extrahierte mit Dichlormethan, wusch mit Wasser und trocknete über MgS0₄. Nach Abdampfen des Lösungsmittels i.Vak. und Chromatographie des Rückstands an 100 g Kieselgel (Toluol: Ethylacetat 1:9) erhielt man 74 mg (8,2 %) 4-[3(R)-Benzoylamino-2-azetidinon-4(R)-yloxy]-2-butin-1-ol als farblosen Hartschaum.

R = 0,21 (Toluol:Ethylacetat 1:9).

IR (CHC1₃): 3351 (NH, OH), 1779 (C=O, B-Lactam), 1664 und 1528 cm⁻¹ (Amid).

¹_{H}-_{NMR} (200 MHz, DMSO) δ 4,11 (d, J= 6 Hz, 2H, CH₂OH), 4,32 (AB, J= 17 Hz, 2H, CH₂0), 4,65 (dd, J= 9 Hz, 1Hz, 1H, H-3), 5,21 (d, J= 1Hz, H-4), 5,21 (t, J= 6 Hz, CH₂0H) Zus. 2H, 7,5-7,6 (m, 3H, Ph), 7,75 (m, 2H, o-Benzoyl-H), 9,02 (s, 1H, NH), 9,18 (d, J= 9 Hz, 1H, NH).

### Beispiel 18

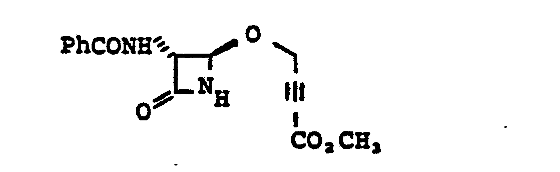

### 4-[3(R)-Benzoylamino-2-azetidinon-4(R)-xyloxy]-2-butin- säure-methylester

a) Eine Suspension von 4,70 g (25 mmol) (1R, 5S)-3-Phenyl-4-oxa-2,6-diazabicyclo[3.2.0]hept-2-en-7-on und 14,3 g (125 mmol - 5 equiv.) 4-Hydroxy-2-butin- säuremethylester (R.A. Earl, Organic Syntheses 60, 81) in 40 ml wasserfreiem Dichlormethan wurde bei Raumtemp. mit 0,41 ml Bortrifluoridetherat versetzt. Man rührte 1 h bei Raumtemperatur, wobei eine klare Lösung entstand. Danach wurde in verdünnte NaHCO₃-Lösung gegossen, man extrahierte mit Dichlormethan, wusch die Extrakte mit Wasser und trocknete über MgSO₄. Nach Abdampfen des Lösungsmittels i.Vak. und Filtration des Rückstands an 120 g Kieselgel (Toluol: Ethylacetat 2:3) erhielt man 4,74 g (63 %) 4-[3(R)-Benzoylamino-2-azetidinon-4(R)-yloxy]-2-butin- säuremethylester als farblose Kristalle, Schmp.: 142-143°C, R_{F}: 0,29 (Toluol:Ethylacetat 2:3), [α]²⁰ _{D} = 18.37° (1.0345 proz. in Aceton).

IR (CHC1₃): 2240 (C⁼C), 1784 (C=O, ß-Lactam, 1717 (C=_{O}, Ester), 1662 cm⁻¹ (_{C}=_{O}, Amid).

¹H-NMR (250 MHz, DMSO) δ 3,70 (s, 3H, COOCH₃), 4,54 (AB, J= 15 Hz, 2H, CH₂0), 4,66 (dd, J= 8 Hz, 1 Hz, 1H, H-3), 5,24 (d, J= 1 Hz, 1H, H-4), 7,5-7,65 (m, 3H, Ph), 7,9 (m, 2H, ortho-Benzoyl-H), 9,08 (s, 1H, NH), 9,18 (d, J= 8 Hz, 1H, NH).

^{C}₁₅^{H}₁₄^{N}₂0₅ (302.3) Ber.: C 59,60 H 4,67 _{N 9},₂₇ Gef.: 59,2 4,7 9,2

b) Eine Suspension von 94 mg (0,5 mmol) (1R, 5S)-3-Phenyl-4-oxa-2,6-diazabicyclo[3.2.0]hept-2-en-7-on und 290 mg (2,5 mmol) 4-Hydroxy-2-butinsäuremethyl- ester in 0,8 ml wasserfreiem Dichlormethan wurde bei 0°C mit 114 mg (0,6 mmol - 1,2 equiv.) wasserfreiem Zinn(II)chlorid versetzt. Anschließend wurde 1 h bei Raumtemperatur gerührt, wobei eine klare Lösung entstand. Nach Aufarbeitung und Reinigung wie unter a) beschrieben, erhielt man 35 mg (23 %) der Titelverbindung vom Schmp.: 142-142,5°C, deren physikalische Daten mit der nach Methode a) hergestellten Substanz identisch waren.

### Beispiel 19

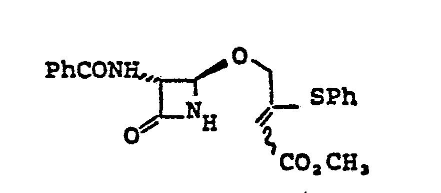

### E,Z-4[3(R)-Benzoylamino-2-azetidinon-4(R)-yloxy%-3-phenylthio-2-butensäuremethylester

Eine auf 0°C gekühlte Lösung von 302 mg (1 mmol) 4-[3(R)-Benzoylamino-2-azetidinon-4(R)- yloxy7-2- butinsäuremethylester in 5 ml THF wurde nacheinander mit 113 µl (1,1 mmol) Thiophenol und 153 µl Triethylamin versetzt. Das Eisbad wurde entfernt und die Mischung wurde 3 h bei Raumtemperatur gerührt. Dann wurde in gesättigte NaHCO₃-Lösung gegossen, mit Dichlormethan extrahiert,mit Wasser gewaschen und über MgS0₄ getrocknet. Das Lösungsmittel wurde i.Vak. abgedampft und das Rohprodukt aus Dichlormethan-Ether

kristallisiert. Man erhielt 280 mg (68 %) der Titelverbindung als Gemisch der Doppelbindungsisomere; Schmp.: 178°C, R_{F}: 0,48 (Toluol:Ethylacetat 1:4).

IR (KBr): 3317 (NH), 1807 (C=O, B-Lactam), 1704 (C=O, Ester), 1642 und 1533 (Amid), 1602 cm⁻¹ (_{S}-_{C}=_{C}).

¹_{H}-_{NMR} (250 _{MH}z, DMSO) δ 3,61, 3,63 (s, 3H, COOCH₃), 3,95 (s, 2H, CH₂0), 4,48 (d, J= 8 Hz, 1H, H-3), 4,78 (s, 1H, H-4), 6,14, 6,16 (s, 1H, -CH=_{C}), 7,3-7,6 (m, 8H, pH), 7,85 (m, 2_{H}, o-Benzoyl-H), 8,85 (s, 1H, NH-B-Lactam), 9,05, 9,10 (d, J= 8 Hz, 1H, PhCONH).

^{C}₂₁^{H}₂₀^{N}₂⁰₅^{S} (⁴12.5) Ber.: C 61,15 H 4,89 N 6,79 _{S} 7,77 Gef.: 61,1 4,9 6,8 7,7.

### Beispiel 20

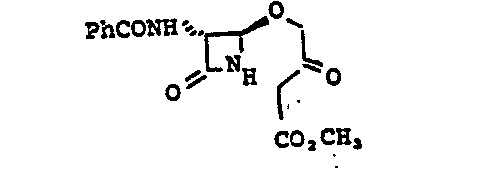

### 4-[3(R)-Benzoylamino-2-azetidinon-4(R)-yloxy]acetessig- säuremethylester

Zu einer auf 0°C gekühlten Lösung von 412 mg (1 mmol) E,Z-4[3(R)-Benzoylamino-2-azetidinon-4(R)-yloxy]-3-phenylthio-2-butensäuremethylester in 13 ml Dioxan:Wasser 10:1 wurden auf einmal 690 mg (5 mmol) N-Bromacetamid gegeben. Die hellbraune Lösung wurde 2 h bei 0°C gerührt und bei dieser Temperatur mit 12 ml gesättigter Na₂SO₃-ZÖsung gequencht. _{M}an rührte noch 40 min bei 0°C, ließ kurz auf Raumtemperatur kommen und extrahierte die Mischung 3 x mit Ethylacetat. Man wusch mit Wasser, trocknete über MgSO₄ und reinigte das Rohprodukt durch Chromatographie an 7,5 g Kieselgel (Toluol:Ethylacetat 1:4). Man erhielt die Titelverbindung als Schaum, R_{F}: 0,22 (Toluol:Ethylacetat 1:4).

IR (CHC1₃): 3328 (NH), 1777 (C=O, B-Lactam), 1737 (C=O, Keton), 1725 (C=O, Ester), 1658 und 1525 cm⁻¹ (Amid).

¹H-NMR (200 MHz, CDCl₃) δ 3,53 (s, 2H, COCH₂COOR), 3,71 (s, 3H, COOCH₃), 4,46, 4,58 (AB, J= 17 Hz, 2H, CH₂0), 4,77 (d, J= 7,5 Hz, 1H, H-3), 5,14 (s, 1H, H-4), 7,4-7,7 (m, 7H, Ph, NH).

### Beispiel 21

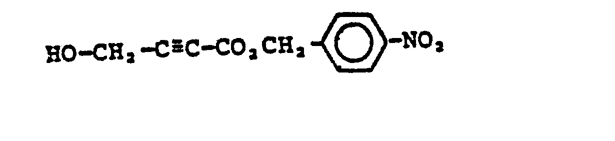

### 4-Hydroxy-2-butinsäure-4-nitrobenzylester

Zu 7,8 ml (23,2 mmol) einer 3 molaren Lösung von Ethylmagnesiumbromid in Ether wurde innerhalb von 30 min bei Raumtemperatur eine Lösung von 3,3 ml (23,2 mmol) Tetra- hydro-2-(2-propinyloxy)-2H-pyran [R.A. Earl et al., Organic Syntheses 60, 81 (198117 in 23 ml wasserfreiem THF getropft. Anschließend wurde 1,5 h bei Raumtemperatur nachgerührt. Diese Lösung wurde innerhalb von 1,5 h zu einer gut gerührten, auf -20°C gekühlten Lösung von 5,0 g (23,2 mmol) Chlorameisensäure-4-nitrobenzylester in 25 ml THF getropft.Anschließend wurde 30 min bei -15°C und 1,5 h bei 0°C nachgerührt, dann durfte die Mischung 12 h bei 0°C stehen, wobei die Magnesiumsalze auskristallisierten. Die Salze wurden unter Feuchtigkeitsausschluß abfiltriert und 2 x mit etwas Toluol gewaschen. Die Filtratlösung wurde 5 x mit gesättigter NaCl-Lösung gewaschen und über MgS0₄ getrocknet. Das Lösungsmittel wurde i.Vak. abgedampft, der Rückstand in 25 ml wasserfreiem Methanol gelöst, mit 1 ml Ionenaustauscher Dowex-50-X4 (H⁺-Form, mit wasserfreiem Methanol vorgespült) 1 h bei Raumtemperatur gerührt. Der Ionenaustauscher wurde abgetrennt, die Filtratlösung i.Vak. eingeengt und schließlich i. Hochvak. getrocknet. Die Behandlung mit Ionenaustauscher wurde wie oben beschrieben wiederholt. Das Rohprodukt wurde an 300 g Kieselgel (Toluol:Ethylacetat 4:1) chromatographiert und man erhielt 2,07 g (38 %) 4-Hydroxy-2-butinsäure-4-nitrobenzylester als farblose Kristalle, Schmp.: 93-94°C, R_{F}: 0,27 (Toluol:Ethylacetat 4:1).

IR (KBr): 3475 (OH), 2235 (-C≡C-), 1690 (C=O, Ester) 1524 (NO₂, as.), 1348 cm⁻¹ (NO₂, sym.).

¹H-NMR (200 MHz, DMSO) δ 4,30 (d, J= 6 Hz, 2H, CH₂OH), 5,38 (s, 2H, CH₂-), 5,63 (t, J= 6 Hz, 1H, CH₂0H), 7,68 (d, _{J}= _{20 Hz}, 2_{H}, Hₐᵣₒₘ.)' 8,15 (_{d}, J= 20 Hz, 2H, Hₐᵣₒₘ.).

C₁₁H₉NO₅ (235.2) Ber.: C 56,2 H 3,9 _{N} 6,0 Gef.: 56,2 3,9 6,1

### Beispiel 22

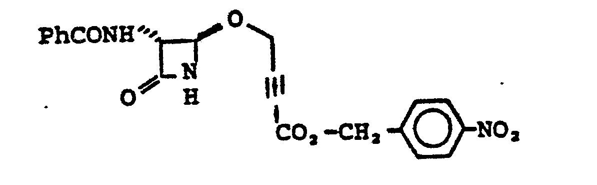

### 4-[3(R)-Benzoylamino-2-azetidinon-4(R)-yloxy]-2-butin- säure-4-nitrobenzylester

Zu einer gerührten Suspension von 376 mg (2 mmol) (1R, 5S)-3-Phenyl-4-oxa-2,6-diazabicyclo[3.2.0]hept-2-en-7- on und 941 mg (4 mmol) 4-Hydroxy-2-butinsäüre-4-nitrobenzylester in 8 ml wasserfreiem THF wurden bei Raumtemperatur 10 µl Bortrifluoridetherat gespritzt. Nach kurzer Zeit entstand eine klare, hellgelbe Lösung, welche 0,5 h bei Raumtemperatur gerührt wurde. Danach wurde in verdünnte NaHCO₃-LÖsung gegossen, mit Dichlormethan extrahiert, mit Wasser gewaschen und über MgS0₄ getrocknet. Nach Abdampfen des Lösungsmittels i.Vak. und Chromatographie des Rückstands an 60 g Kieselgel (Toluol: Ethylacetat 35:65) erhielt man 557 mg (66 %) 4-[3(R)-Benzoylamino-2-azetidinon-4 (R)-yloxy]-2-butinsäure-4-nitrobenzylester als farblosen Hartschaum. R_{F}: 0,55 (Ethylacetat), [α]²⁰ _{D}= -35.28° (0,5 proz. in CHCl₃).

IR (KBr): 3330 (NH), 2244 (C=C), 1780 (C=O, B-Lactam), 1715 (C=O, Ester), 1651 und 1524 (Amid), 1524 (N0₂-as.), 1350 cm⁻¹ (N0₂-sym.).

¹H-NMR (250 MHz, CDCl₃)δ 4,54 (AB, J= 16 Hz, 2H, CH₂0), 4,69 (d, J= 9 Hz, 1H, H-3), 5,28 (s, 2H, CH₂-Benzyl), 5,34 (s, 1H, H-4), 7,08 (s, 1H, NH), 7,4-7,6, 7,8 (m, Ph, NH), 7,51, 8,21 (d, J= 9,5 Hz, AB-4-Nitrobenzyl) Zus. 10 H.

C₂₁H₁₇N₃O₇ (423.39) Ber.: C 59,58 H 4,05 _{N} 9,₉₂ Gef.: 58,8 4,0 9,8.

### Beispiel 23

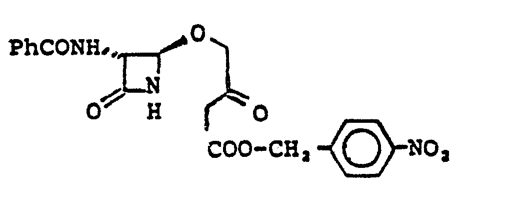

### 4[3(R)-Benzoylamino-2-azetidinon-4(R)-yloxy]-acetessig- säure-4-nitrobenzylester

Eine auf 0°C gekühlte Lösung von 551 mg (1,3 mmol) 4-[3(R)-Benzoylamino-2-azetidinon-4-(R)-yloxy]-2-butinsäure-4-nitrobenzylester in 6,6 ml THF wurde nacheinander mit 0,15 ml (1,43 mmol - 1,1 equiv.) Thiophenol und 0,2 ml (1,43 mmol - 1,1 equiv.) Triethylamin versetzt. Man rührte 3 h bei Raumtemperatur, goß in eine gesättigte NaHC0₃-Lösung, extrahierte mehrmals mit Dichlormethan und trocknete über MgS04.

Das Lösungsmittel wurde i.Vak. abgedampft und der Rückstand in 15 ml Dioxan:Wasser 10:1 gelöst. Bei 0°Cawurden auf einmal 1,44 g (10,41 mmol - 8 equiv.) N-Bromacetamid zugegeben und man rührte 5,5 h bei dieser Temperatur. Danach wurde mit 15 ml kalter, gesättigter Na₂SO₃-LÖsung versetzt und 40 min bei 0°C nachgerührt. Danach durfte sich die Mischung kurz auf Raumtemperatur erwärmen, dann wurde mit Ethylacetat 4 x extrahiert. Die organischen Extrakte wurden mit Wasser.gewaschen und über MgS0₄ getrocknet. Das Lösungsmittel wurde i.Vak. abgedampft, der Rückstand mit wenig Dichlormethan versetzt und nach der Kristallisation mit Ether zerrieben. Man erhielt 479 mg (83 %) der Titelverbindung als farblose Kristalle, Schmp.: 138-139°C, R_{F}: 0,27 (Toluol:Ethylacetat 1:4).

IR (KBr): 3338 (NH), 1174 (C=O, ß-Lactam, 1740 (C=O, Keton), 1720 (C=O, Ester), 1641 und 1512 (Amid), ₁₃46 em⁻¹ NO₂-sym.).

¹H-NMR (200 MHz, DMSO) δ 3,75 (s, 2H, COCH₂COOR), 4,43 (s, 2H, CH₂0), 4,65 (d, J= 9 Hz, 1H, H-3), 5,18 (s, 1H, H-4), 5,30 (s, 2H, CH₂-Benzyl), 7,45-7,9 (m, Ph), 7,65, 8,25 (d, J= 9,5 Hz, AB-4-Nitrobenzyl) Zus. 9H, 9,00 (s, 1H, NH), 9,14 (d, J= 9 Hz, 1H, PhCONH).

C₂₁H₁₉N₃O₈ (⁴⁴1.4) Ber.: C 57,14 H 4,34 _{N} 9,52 Gef.: 56,9 4,3 9,2.

### Beispiel 24

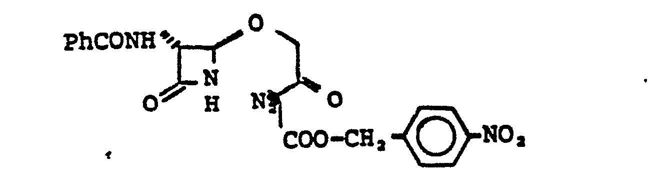

### 4-[3(R)-Benzoylamino-2-azetidinon-4(R)-yloxy]-2-diazo-3-oxo-butansäure-4-nitrobenzylester

Zu einer auf 0°C gekühlten Suspension von 662 mg (1,5 mmol) 4-[3(R)-Benzoylamino-2-azetidinon-4(R)-yloxy]-acet- essigsäure-4-nitrobenzylester und 395 mg (1,74 mmol - 1,16 equiv.) 4-Carboxybenzolsulfonylazid in 15 ml wasserfreiem Acetonitril tropfte man 0,75 ml (5,4mmol - 3,6 equiv.) Triethylamin. Das Kühlbad wurde entfernt und man rühte 1 h bei Raumtemperatur nach. Anschließend wurde mit 100 ml Ethylacetat versetzt, der unlösliche Niederschlag abgesaugt und verworfen. Die Filtratlösung wurde i.Vak. eingedampft und der Rückstand mit Ether versetzt. Man erhielt 540 mg (77 %) der Titelverbindung als farblose Kristalle, Schmp.: 135°C, R_{F}: 0,44 (Toluol:Ethylacetat 1:9).

IR (KBr): 3317 (NH), 2154 (N₂), 1789 (C=O, B-Lactam), 1709 (C=0, Ester, 1660 und 1521 cm⁻¹ (Amid).

¹H-NMR (200 MHz, DMSO) δ 4,64 (dd, J= 9 Hz, 1 Hz, 1H, H-3), 4,72 (s, 2H, CH₂0), 5,27 (d, J= 1 Hz, 1H, H-4), 5,44 (s, 2H, CH₂-Benzyl). 7,5-7,9 (m,Ph) 7,71, 8,26 (d, J= 9,5 Hz, AB-4-Nitrobenzyl) Zus. 9H, 9,00 (s, 1H, NH), 9,15 (d, J= 9 Hz, 1H, PhCONH).

C₂₁H₁₇N₅O₈ (467.39) Ber.: C 54,0 H 3,7 N 15,0 Gef.: 53,6 3,8 14,8.

### Beispiel 25

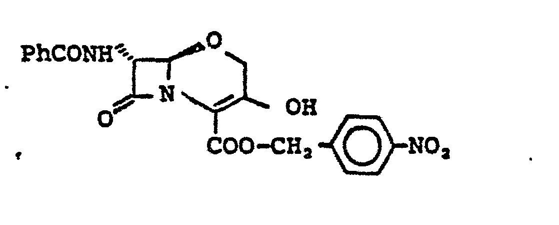

### (6R, 7R)-7-Benzoylamino-3-hydroxy-8-oxo-5-oxa-1-azabi- cyclo[4.2.0]oct-2-en-2-carbonsäure-4-nitrobenzylester

Wie für Beispiel 5 beschrieben erhielt man aus 510 mg (1,1 mmol) 4[3(R)-Benzoylamino-2-azetidinon-4(R)-yloxy]-2-diazo-3-oxo-butansäure-4-nitrobenzylester nach Kristallisation des Rohprodukts aus Ether 230 mg (47,6 %) der Titelverbindung, R_{F}: 0,37 (Ethylacetat).

IR (KBr): 3320 (b, OH, NH), 1776 (C=0, B-Lactam), 1652 (ß-Ketoester, Enolform), 1660 und 1525 cm⁻¹ (Amid).

¹H-NMR (200 M_{H}z, DMSO) δ 4,38 (AB, J= 18 Hz, 2H, CH₂0), 4,98 (d, J= 9 Hz, 1H, H-7), 5,20 (s, 1H, H-6), 5,45 (s, 2H, CH₂-Benzyl), 7,5-8,2 (m, 9^{H,} Hₐᵣₒₘ), 9,24 (d, J= 9 Hz, 1H, NH).

### Beispiel 26

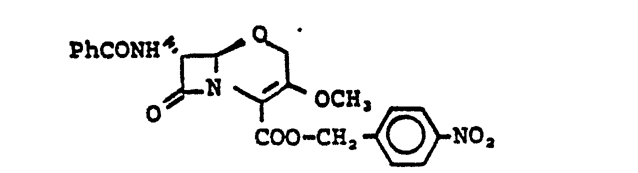

### (6R, 7R)-7-Benzoylamino-3-methoxy-8-oxo-5-oxa-1-azabi- cyclo[4.2.0]oct-2-en-2-carbonsäure-4-nitrobenzylester

Wie für Beispiel 6 beschrieben erhielt man aus 130 mg (0,3 mmol) (6R, 7R)-7-Benzoxylamino-3-hydroxy-8-oxo-5-oxa-1-azabicyclo[4.2.0]oct-2-en-2-carbonsäure-4-nitrobenzylester nach Cromatographie des Rohprodukts 15 g Kieselgel (Toluol:Ethylacetat 35:65) 75 mg (56 %) der Titelverbindung als farbloses Pulver, R_{F}: 0,33 (Toluol:Ethylacetat 3:7).

IR (KBr): 3361 (NH), 1771 (C=O, B-Lactam), 1724 (C=O, Ester), 1650 und 1517 (Amid), 1605 (C=C), 1349 cm⁻¹ (NO₂-sym.).

¹H-NMR (200 MHz, CDCl₃) δ 3,85 (s, 3H, OCH₃), 4,50, 4,63 (AB, J= 17 Hz, 2H, CH₂0), 4,97 (d, J= 8 Hz, 1H, H-7), 5,10 (s, 1H, H-6), 5,30, 5,45 (AB, J= 15 Hz, CH₂-Benzyl), 7,11 (d, J= 8 Hz, 1H, NH), 7,4-7,9 (m, Ph), 7,65, 8,24 (AB, J= 10 Hz, 4-Nitrobenzyl), Zus. 9H.

### Beispiel 27

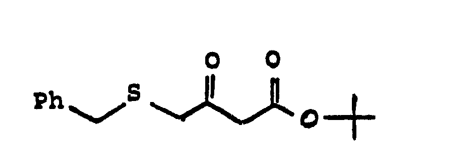

### 4-Benzylthioacetessigsäure-tert.-butylester

Zu einer Suspension von 1,8 g (60 mmol) Natriumhydrid (80 % in Paraffinöl)in 50 ml wasserfreiem THF wurden bei Raumtemperatur langsam 7,04 ml (60 mmol) Benzylmercaptan getropft. Dann wurde 15 Min. nachgerührt und anschließend auf 0°C abgekühlt. Bei dieser Temperatur gab man innerhalb von 1 h eine Lösung von 10,57 g (55 mmol) 4-Chlor- acetessigsäure-tert.-butylester in 20 ml THF zu. Das Eisbad wurde entfernt, man rührte noch 1 h bei Raumtemp. und neutralisierte die Mischung durch Zugabe einiger Tropfen 10 %iger HCl. Das Lösungsmittel wurde i.Vak. abgedampft, man versetzte den Rückstand mit Ether und Wasser, extrahierte mehrmals mit Ether, wusch die Extrakte mit Wasser und trocknete über MgS0₄. Abdampfen des Ethers i.Vak. ergab ein öl, welches durch Chromatographie an 800 g Kieselgel (Toluol) gereinigt wurde. Man erhielt 12,74 g (83 %) der Titelverbindung in Form eines farblosen öls. R_{F},: 0,13 (Toluol).

IR (CHC1₃) 1740-1710 cm⁻¹ (C=0, ß-Ketoester).

¹H-NMR (200 MHz, CDCl₃) δ 1,49 (s. 9H, C(CH₃)₃), 3,22 (s. 2H, CH₂), 3,52 (s. 2H, CH₂). 3,68 (s. 2H, CH₂), 7,33 (s. 5H, Ph).

C₁₅H₂O₃S (280,4) Ber.: C 64,3 H 7,2 S 11,4 Gef.: 64,3 7,2 11,3.

### Beispiel 28

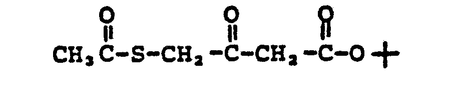

### 4-Acetylthioacetessigsäure-tert.-butylester

Zu einer auf 0°C abgekühlten Lösung von 3,85 g (20,0 mmol) 4-Chloracetessigsäure-tert.-butylester in 40 ml wasserfreiem Acetonitril gab man 2,40 g (21,0 mmol) trockenes Kaliumthioacetat und rührte 30 Min. bei dieser Temperatur. Danach goß man die Mischung in ein Gemisch aus NaCl-Lösung und Ethylacetat, trennte die organische Phase ab, extrahierte mit 2 x 30 ml Ethylacetat, wusch mit Wasser und trocknete über MgS0₄. Nach Abdampfen des Lösungsmittels i.Vak. und Chromatographie des Rückstandes an 60 g Kieselgel (Toluol:Ethylacetat 95:5) erhielt man 3,87 g (83 %) der Titelverbindung als farbloses Ö1, R_{F}: 0,24 (Toluol:Ethylacetat 95:5).

I_{R} (CHCl₃) ₁₇₄₀-₁₇₁₀ cm⁻¹ (C=O) .

¹H-NMR (250MHz, CDC1₃) 1,50 (s. 9H, C(CH₃)₃, 2,42 (s. 3H, CH₃CO₃), 3,53 (s. 2H, CH₂), 3,90 (s, 2H, CH2).

### Beispiel 29

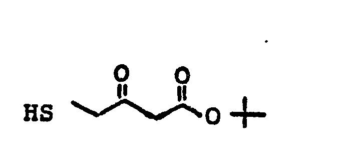

### 4-Mercaptoacetessigsäure-tert.-butylester

Eine Lösung von 465 mg (2 mmol) 4-Acetylthioacetessig- säure-tert.-butylester in 4 ml wasserfreiem Methanol, wurde mit 45 Tropfen einer 1 N Lösung von Natriummethylat in Methanol versetzt und bis zur völligen Umsetzung (DC-Kontrolle) bei Raumtemperatur unter Stickstoff gerührt. Danach wurde das Methanol i.Vak. abgedampft und der Rückstand an 40 g Kieselgel (Toluol:Ethylacetat 94:6)durch "flash-Chromatographie" gereinigt. Man erhielt 157 mg (41 %) der Titelverbindung als farbloses öl, Rp: 0,13 (Toluol:Ethylacetat 94:6).

IR (CHCl₃) 3480, 2986, 1712, 1152 cm⁻¹.

NMR (250 MHz, CDC1₃) δ 1,5 (s. 9H, C(CH3)3), 2,6-3,8 (m, 5H).

Sdp. ca. 150°C/0,5 mm Mikrokugelrohr. C₈H₁₄O₃S (190.3) Ber.: C 50.50 H 7.₄₂ Gef.: C 50.6 H 7.4

Die in Beispiel 29 erhaltene Verbindung wurde wie in der Reaktionsfolge der Beispiele 3, 4, 5, 11, 12, 13 und 14 beschrieben zu (6R, 7S)-7-[(2R)-2-tert.-Butoxycarbonyl- amino-2-(4-tert.-butoxycarbonylaminophenyl)acetamido]-3-chloro-8-oxo-5-thia-l-azabicyclo [4.2.9]oct-2-en-2-carbonsäure-tert.-butylester umgesetzt.

### Beispiel 30

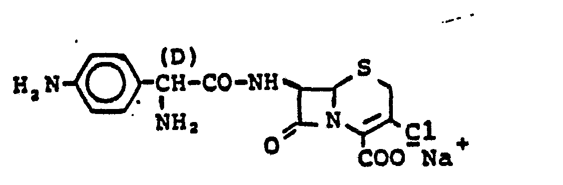

Natrium-(6R, 7R)-7-((2R)-2 Amino-2-(4-aminophenyl)-acet- amido]-3-chloro-8-oxo-5-thia-1-azabicyclo[4.2.0]oct-2-en-2-carboxylat

Wie für Beispiel 10 beschrieben erhielt man aus 2,08 g (2,78 mmol) (6R, 7R)-7-[(2R)-2-tert.-Butoxycarbonylamino-2-(4-tert.-butoxycarbonylaminophenyl)acetamido]-3-chloro-8-oxo-5-thia-1-azabicyclo[4.2.0]oct-2-en-2-carbonsäure- tert.-butylester 768 mg (72 %) der Titelverbindung in einer Reinheit von 87 % (HPLC).

IR (KBr): 3500-2800 (b), 1770 (C=O, B-Zactam), 1680 und 1520 (Amid), 1600 cm⁻¹ (COONa).

¹H-NMR (250 MHz, DCO₂D) 3,50, 3,83, (AB, J= 19 Hz, 2H, (CH₂S), 5,20, (d, J= 4,5 Hz, 1H, H-6), 5,60 (s, 1H, CHCO), 5,83 (d, J= 4,5 Hz, 1H, H-7), 7,71, 7,78 (AB, J= 7 H^{z, 2H,} Hₐᵣₒₘ).

### Beispiel 31

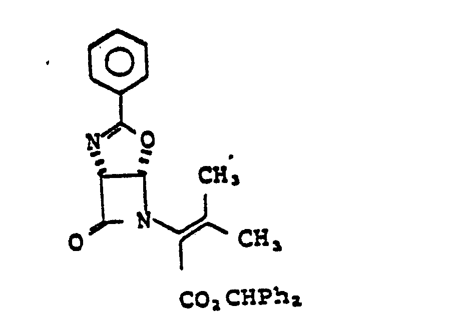

Eine Lösung von 4,53 g (10,0 mmol) Diphenylmethyl-2-[(1R,5S)-3-phenyl-7-oxo-4-oxa-2,6-diazabicyclo[3.2.0]-hept-2-en-6-yl17-3-methylbut-3-enoat in 250 ml wasserfreiem Dichlormethan wurde mit 558 µl (4,0 mmol) Triethylamin versetzt und 6 h bei Raumtemperatur gerührt. Anschließend wurde in 300 ml eiskalte 1 N HCl gegossen, mit 2 x 50 ml Dichlormethan extrahiert, mit 250 ml gesättigter NaHCO₃-Lösung und Wasser gewaschen und über MgSO₄ getrocknet. Nach Abdampfen des Lösungsmittels im Vakuum und Chromatographie des Rückstandes an 300 g Kieselgel (Toluol:Ethylacetat 85:15) erhielt man 3,81 g (84 %) Diphenylmethyl-2-L(1R,5S)-3-phenyl-7-oxo-4-oxa-2,6-diazabicyclo[3.2.0]hept-2-en-6-yl]-3-methylbut-2-enoat als farblosen Hartschaum, R_{F} 0,54 (Ether).

IR (KBr): 1783 (C=_{O}, B-Lactam), 1722 (C=O, Ester), ₁₆₃₂ cm⁻¹ (C=N) .

¹H-NMR (200 MHz, CDCl₃) : δ = 1,83 (s. 3H,CH₃), 2,28 (s. 3H,CH₃), 5,40 (s, J= 4 Hz; 1H H-5), 6,08 (d, J= 4 Hz; 1H, H-1), 6,95 (s, 1H, COCHPh₂), 7,3 - 7,6 (m, 13H, C₆H₅), 7,95 (m, 2H, o-Phenyl-H).

C₂₈H₂₄N₂O₄ (452,5) Ber.: C 74,32 H 5,32 _{N} 6,₁₉ Gef.: C 74,0 H 5,3 N 6,2

### Beispiel 32

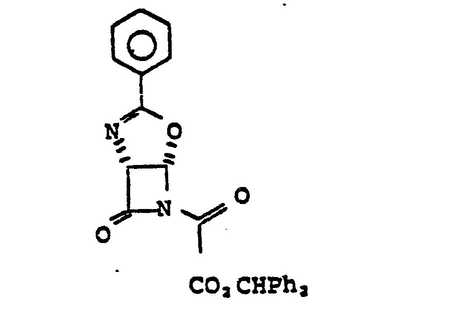

Durch eine auf -70°C gekühlte Lösung von 1,50 g (3,32 mmol) Diphenylmethyl-2-[(1R, 5S)-3-phenyl-7-oxo-4-oxa-2,6-diaza- bicyclo [3.2.0]hept-2-en-6-yl)]-3-methyl-2-enoat in 50 ml wasserfreiem Dichlormethan wurde bis zur Blaufärbung ein Ozon-Sauerstoffgemisch geleitet. Anschließend wurde 10 Min. mit Stickstoff gespült um überschüssiges Ozon zu entfernen, dann wurden bei -70°C 1,95 ml (26,52 mmol) Dimethylsulfid zugegeben. Es wurde 30 Min. bei -10°C und 1 h bei Raumtemperatur gerührt und dann das Lösungsmittel im Vakuum abgedampft. Der Rückstand wurde in 100 ml Dichlormethan aufgenommen, mit gesättigter NaHCO₃-LÖsung und Wasser gewaschen und über MgSO₄ getrocknet. Die organische Phase wurde im Vakuum eingeengt, mit etwas Chloroform aufgenommen,mit Ether versetzt und stehen gelassen. Man erhielt 989 mg (70 %) Diphenylmethyl-2-[(1R, 5S)-3-phenyl-7-oxo-4-oxa-2,6-diazabicyclo[3.2.0]hept-2-en-6-yl]-2-oxo-acetat als farblose Kristalle, Schmp. 182°C, R_{F} 0,37 (Ethylacetat:Hexan 1:1) - [Zersetzt sich bei Kontakt zu Kieselgel]-[α]²⁰ _{D} 35,5° (1,006 _{%} in Chloroform).

IR (KBr): 1817 (C=O, B-Lactam), 1754 (C=O, Ester), 1712 (C=O, Amid), 1640 cm⁻¹ (_{C}=_{N}).

¹H-NMR (200 MHz, CDCl₃) : δ= 5,55 (d, J= 4,5 Hz; 1H, H-5), 6,54 (d, J= 4,5 Hz; 1H, H-1), 7,07 (s, 1H, COOCHPh₂) , 7,2-7,6 (m, 13H, C₆H₅) , 7,9 (m, 2H, o-Phenyl-H).

MS (70 eV): m/e = 426 (M⁺); ber. 426,4.

C₂₅H₁₈N₂O₅ (426,4) Ber. C 70,42 H 4,25 N 6,57 Gef. C 70,4 H 4,3 N 6,7

### Beispiel 33

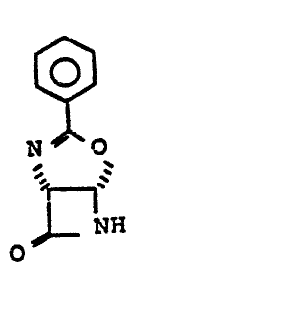

Eine Suspension von 552 mg (1,29 mmol) Diphenylmethyl-2-[(1R, 5S)-3-phenyl-7-oxo-4-oxa-2,6-diazabicyclo[3.2.0]-hept-2-en-6-yl17-2-oxo-acetat in 108 ml wasserfreiem Methanol wurde mit 36 ml einer 0,002 %igen Lösung von Natriummethanolat in Methanol versetzt und 15 Min. bei Raumtemperatur gerührt. Anschließend gab man 21 µl Eisessig und dampfte das Methanol im Vakuum ab. Der Rückstand wurde in 100 ml Dichlormethan gelöst, mit gesättigter NaHCO₃-Läsung und Wasser gewaschen und über MgSO₄ getrocknet. Nach Abdampfen des Lösungsmittels im Vakuum und Chromatographie des Rückstandes an 20 g Kieselgel (Ethylacetat:Hexan 6:4) erhielt man 112 mg (1R, 5S)-3-Phenyl-7-oxo-4-oxa-2,6-diazabicyclo[3.2.0]-hept-2-en als farblose Kristalle, Schmp. 169°C, R_{F}:0,23 (Ether), [α]₂₀^{D}= -114,8° (0,836 % in Aceton).

IR (KBr): 1772 (C=O, B-Lactam), 1616 cm⁻¹ (C=O, C=_{N}).

¹H-NMR (200 MHz, DMSO): δ= 5,35 (dd, J= 4 Hz, J= 4 Hz; 1H, H-5), 6,11 (d, J= 4 Hz; 1H, H-1), 7,5-7,7 (m, 3H, C6H5), 7,93 (m, 2H, o-C₆H₅), 9,36 (d, J= 4 Hz; 1H, NH).

### Beispiel 34

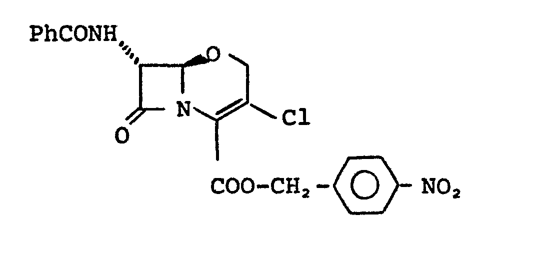

### (6_{R},7_{R})-7-Benzoylamino-3-chloro-8-oxo-5-oxa-1-azabicyclo [4.2.0]oct-2-en-2-carbonsäure-4-nitrobenzylester

Wie für Beispiel 11 beschrieben, erhielt man aus 4.39 g (10 mmol) (6R,7R)-7-Benzoylamino-3-hydroxy-8-oxo-5-oxa-1-azabicyclo[4.2.0]oct-2-en-2-carbonsäure-4-nitrobenzylester und 1.40 ml (16 mmol) Phosphortrichlorid 1.22 g (26%) der Titelverbindung als farblose Kristalle, Schmp. 192°C, R_{F}: 0.35 (Toluol:Ethylacetat 7:3).

IR(KBr): 1791 (C=₀, B-Lactam), 1733 (C=O, Ester), 1642 und 1516 (Amid), 1346 cm⁻¹ (NO₂ , sym.).

¹H-NMR(250 MHz, CDCl₃-DMSO) δ 4.48, 4.54 (AB, J=17Hz, 2H, CH₂0), 4.99 (d, J=7.5Hz, 1H, H-7), 5.33 (s, 1H, H-6), 5.43, 5.54 (AB, J=13_{H}z, 2H, CH₂-Benzyl), 7.4-7.6 (m, 3H, Ph), 6.75 (d, J=10Hz, 2H, PNB), 7.96 (m, 2H, o-Benzoyl-H), 8.24 (d, J=10Hz, 2H, PNB), 9.18 (d, J=7.5Hz, 1H, NH).

C₂₁H₁₆ClN₃O₇ (457.8) Ber.: C 55.09 H 3.52 N 9.18 Gef.: C 55.4 H 3.7 N 9.0

### Beispiel 35

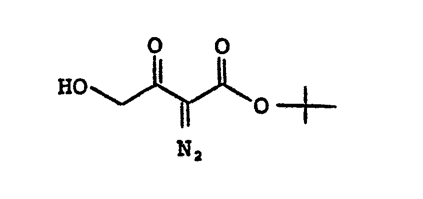

### tert.-Butyl-2-diazo-4-hydroxy-3-oxo-butanoat

Wie für Beispiel 34 beschrieben, erhielt man aus 4.2 g (24.1 mmol) 4-Hydroxyacetessigsäure-tert.-butylester (Beispiel 2) und 5.2 g (26.5 mmol) p-Toluolsulfonsäureazid nach 3 h bei 0°C und 30 min. bei Raumtemperatur und Chromatographie des Rohprodukts an 200 g Kieselgel (Toluol : Ethylacetat 85: 15) 3.43 g (71%) der Titelverbindung als Kristalle, R_{F}: 0,36 (Toluol : Ethylacetat 4:1), Schmp.: 35°C.

IR(CHCl₃): 3495 (OH), 2982,ᵢ2141 (N₂), 1709 (C=O), 1643 (C=O), 1335, ₁₁₃₇, ₉₉₀ cm⁻¹.

¹H-NMR(250 _{MH}z, CDCl₃)δ 1.52 (s, 9H, C(CH₃)₃), 3.48 (t, _{J}=_{5Hz}, 1H, CH₂OH), 4.59 (d, J=5Hz, 2H, CH₂OH).

C₈H₁₂N₂O₄ (200.2) Ber.: C 48.00 H 6.04 Gef.: C 48.0 H 6.1

### Beispiel 36

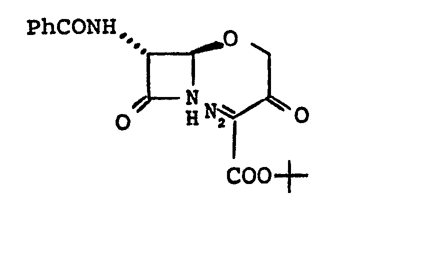

### 4-[3(R)-Benzoylamino-2-azetidinon-4(R)-yloxy]-2-diazo-3-oxo-buttersäure-tert.-butylester

a) Eine auf o°C gekühlte Suspension von 564 mg (3.0 mmol) (1R, 5S)-3-Phenyl-4-oxa-2,6-diazabicyclo[3.2.0]hept-2-en-7-on und 1.08 g (5.4 mmol -1.8 equiv.) tert.-Butyl-2-diazo-4-hydroxy-3-oxo-butanoat in 12 ml wasserfreiem THF wurde tropfenweise mit 90 µl Bortrifluoridetherat versetzt. Das Kühlbad wurde entfernt und nach 10 min. entstand eine klare hellgelbe Lösung. Nach 20 min. wurde in ein Gemisch aus NaHCO₃-LÖsung, Eis und Dichlormethan gegossen. Man extrahierte mit Dichlormethan (3x), wusch mit Wasser und trocknete über Magnesiumsulfat. Nach Abdampfen des Lösungsmittels im Vakuum und Kristallisation des Rückstands aus Dichlormethan-Ether-Pentan erhielt man 861 mg (74%) der Titelverbindung als hellgelbe Kristalle, Schmp.: 98°C , R_{F} = 0.29 (Toluol : Ethylacetat 2:3). Die spektroskopischen Daten waren identisch mit der in Beispiel 4 beschriebenen ; Verbindung.

b) Wie oben beschrieben, erhielt man aus 941 mg (5.0 mmol) (1R, 5S)-3-Phenyl-4-oxa-2,6-diazabicyclo[3.2.0]hept-2-en-7-on und 1.10 g (5.5 mmol - 1.1 equiv.) tert.-Butyl 2-diazo-4-hydroxy-3-oxo-butanoat nach einer Reaktionszeit von 20 min. 1.36g (70%) der kristallinen Titelverbindung, Schmp.: 103°C.

### Beispiel 37

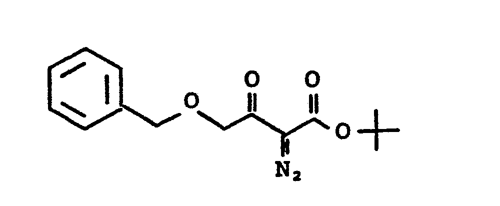

### tert.-Butyl-4-benzyloxy-2-diazo-3-oxo-butanoat

Eine auf 0°C gekühlte Lösung von 5,29 g (20 mmol) 4-Benzyloxy- acetessigsäure-tert.-butylester und 4,34 g (22 mmol) p-Toluolsulfonsäureazid in 40 ml wasserfreiem Acetonitril wurde tropfenweise mit 5,54 ml (40 mmol - 2 equiv.) Triethylamin versetzt. Die Mischung wurde 30 min. bei 0°C gerührt, mit 8 g Kieselgur versetzt, i.Vak. eingedampft und an 100 g Kieselgel (Toluol : Ethylacetat 95:5) chromatographiert. Man erhielt 3,98g(69%) der Titelverbindung als farblose Kristalle, Schmp. : 68°C, R_{F}: 0,31 (Toluol : Ethylacetat 95:5).

IR (KBr) : 2144 (N₂), 1700, 1673 cm⁻¹.

¹H-NMR (300 MHz, CDCl₃) δ 1,51 (s, 9H, C(CH)₃)₃ 4,59 (s, 2H, CH₂), 4,67 (s, 2H, CH₂), 7,3-7,5 (m, 5H, Ph).

C₁₅H₁₈N₂O₄(290,3) Ber. : C 62,06 H 6,23. N 9,65 Gef. : C 62,1 H 6,2 N 9,6.

## Patentansprüche

1. Verfahren zur Herstellung von 7-Acylamino-3-hydroxy-3-cephem-4-carbonsäuren und 7-Acylamino-3-hydroxy-1-dethia-1-oxa-3-cephem-4-carbonsäuren und ihrer Derivate der allgemeinen Formel (1), . 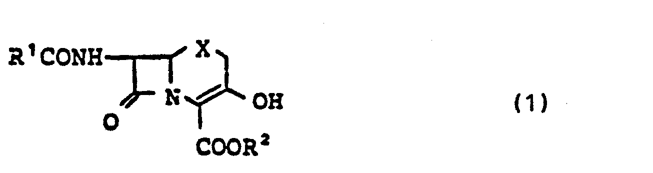 in welcher
R für Wasserstoff oder für gegebenenfalls substituiertes Alkyl, Alkenyl, Alkinyl, Aralkyl, Aryl, Heteroaryl, Heteroaralkyl, Aryloxyalkyl, Heteroaryloxyalkyl, Alkoxyalkyl, Arylthioalkyl, Heteroarylthioalkyl, Alkylthioalkyl, Alkoxy, Aryloxy, Alkylthio oder Arylthio steht,
R² Wasserstoff oder eine Carboxyschutzgruppe oder einen pharmazeutisch verwendbaren Esterrest bedeutet,
und
X für Schwefel oder Sauerstoff steht,
dadurch gekennzeichnet, daß man Verbindungen der allgemeinen Formel (4), 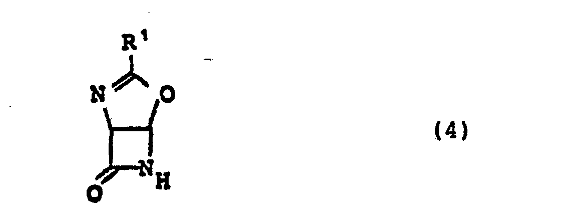 in welcher
R¹ die oben angegebene Bedeutung hat,
a) mit einer Verbindung der allgemeinen Formel (5), 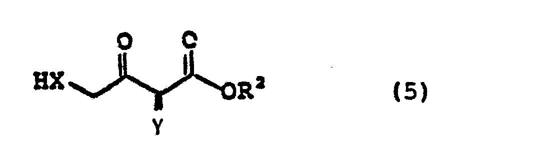 in welcher R² und X die oben angegebene Bedeutung haben und Y für Diazo (N₂) oder Wasserstoff (H₂) steht, in einem inerten Lösungsmittel in Gegenwart eines Lewissäure- oder Protonensäurekatalysators umsetzt, oder
b) mit einer Verbindung der allgemeinen Formel (6) 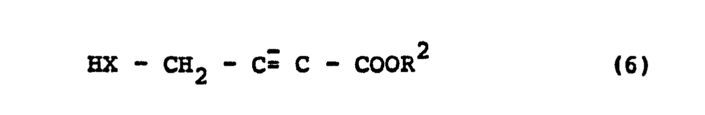 in welcher
_{R}² und X die oben angegebene Bedeutung haben,
wie unter a) beschrieben umsetzt und die Dreifachbindung der dabei entstehenden Zwischenverbindung hydratisiert, die so für Y = Wasserstoff (für Y = Diazo entstehen direkt die Verbindungen der allgemeinen Formel (2)) erhaltenen Verbindungen der allgemeinen Formel (3) 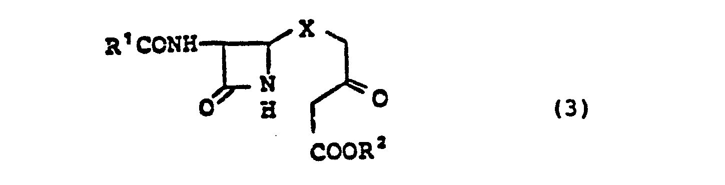 in welcher
_{R}', R² und X die oben angegebene Bedeutung haben,
in einem Lösungsmittel, mit einem Azid, in Ge--genwart einer Base zu Verbindungen der allgemeinen Formel (2) umsetzt und die so erhaltenen Verbindungen der allgemeinen Formel (2), 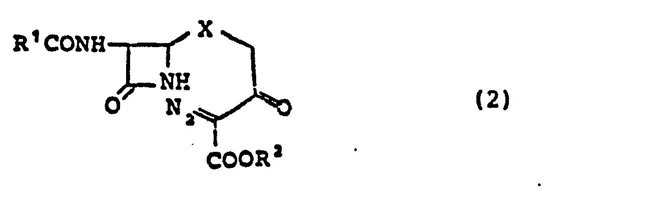 in welcher
R¹, _{R}² und _{X} die oben angegebene Bedeutung haben,
in einem inerten Lösungsmittel, durch Bestrahlen oder durch Erwärmen in Gegenwart eines Katalysators, zu Verbindungen der allgemeinen Formel (1) umsetzt.

2. Verfahren nach Anspruch 1, dadurch gekennzeichnet, daß bei der Umsetzung von Verbindungen der allgemeinen Formel (2) zu Verbindungen der allgemeinen Formel (1) als Katalysatoren Komplexe oder Salze von übergangsmetallen oder elementare übergangsmetalle eingesetzt werden.

3. Verfahren nach Anspruch 2, dadurch gekennzeichnet, daß die Umsetzung in Benzol, Toluol, THF oder Dichlormethan durchgeführt wird.

4. Verfahren nach den Ansprüchen 2 und 3, dadurch gekennzeichnet, daß die Umsetzung bei Temperaturen von 40 - 120°C erfolgt.

5. Verfahren nach Anspruch 1, dadurch gekennzeichnet, daß-die Umsetzung von Verbindungen der allgemeinen Formel (2) zu den Verbindungen der allgemeinen Formel (1) durch Bestrahlen der Verbindungen (2) mit einer Lichtquelle der Wellenlänge ≥ 300 nm erfolgt.

6. Verfahren nach Anspruch 5, dadurch gekennzeichnet, _{d}aß die Umsetzung in Benzol, Toluol, CC1₄, CC1₂, Ether, THF oder Dioxan erfolgt.

7. Verfahren nach Anspruch 1, dadurch gekennzeichnet, daß bei der Umsetzung der Verbindungen der allgemeinen Formel (3) zu Verbindungen der allgemeinen Formel (2) gegebenenfalls substituierte Methan-, Benzol- oder Naphthalinsulfonylazide, insbesondere Toluolsulfonylazid, 4-Dodecylbenzolsulfonylazid und 4-Carboxybenzolsulfonylazid und als Basen Alkalicarbonate, Alkaliamide und organische Amine, insbesondere Triethylamin, Diethylamin, Diisopropylethylamin, Pyridin, Dimethylanilin, 1,5-Diazabcyclo-[5.4.0]-undec-5-en (DBU) bzw. DBN verwendet werden.

8. Verfahren nach Anspruch 1, dadurch gekennzeichnet, daß bei der Herstellung der Verbindungen der allgemeinen Formel (3) durch Verknüpfung der Verbindungen der allgemeinen Formeln (4) und (5) oder (6) als Protonensäuren, Trifluoressigsäure, Trifluormethansulfonsäure, Perchlorsäure, Tetrafluorborwasserstoffsäure, Chlorwasserstoffsäure, Quadratsäure, p-Toluolsulfonsäure, Campfersulfonsäure, Polyphosphorsäure oder als Lewissäuren, Bortrifluorid, Zink(II)chlorid, Aluminiumchlorid, Zinn(IV)chlorid, Quecksilber(II)chlorid, Siliciumtetrachlorid, Zinn-(II)chlorid, Titan(IV)chlorid, Antimon(V)chlorid, Eisen(III)chlorid oder Antimon(III)chlorid; oder Trimethylsilyltrifluormethansulfonat; Trimethylsilyltrifluoracetat, saure Ionenaustauscher oder Kieselgel eingesetzt werden.

9. Verfahren nach Anspruch 1, dadurch gekennzeichnet, daß man die Verbindungen der allgemeinen Formel (5), in denen X und R₂ die angegebene Bedeutung haben, und Y für Diazo (N₂) steht, durch Diazo-Transferreaktionen herstellt.

10. Verfahren nach Anspruch 1, dadurch gekennzeichnet, daß man die Verbindungen der allgemeinen Formel (5), in der X und R₂ die angegebene Bedeutung haben und Y für Wasserstoff (H₂) steht, aus 4-Halogenacetessigsäureestern herstellt.
